(19) Europäisches Patentamt
European Patent Office
Office européen des brevets

(11) EP 0 624 596 B1

(12) EUROPÄISCHE PATENTSCHRIFT

(45) Veröffentlichungstag und Bekanntmachung des Hinweises auf die Patenterteilung:
**11.03.1998 Patentblatt 1998/11**

(51) Int Cl.[6]: **C07J 43/00**, A61K 31/58
// C07J41/00, C07J9/00,
C07J17/00

(21) Anmeldenummer: **94106848.8**

(22) Anmeldetag: **02.05.1994**

(54) **Tetrazolderivate von Gallebnsäuren, Verfahren zu ihrer Herstellung und Verwendung dieser Verbindungen als Arzneimittel**

Tetrazole derivatives of bile acids, a process for their production and their use as medicines

Dérivés tetrazole des acides biliaires, un procédé pour leur production et leur utilisation comme médicaments

(84) Benannte Vertragsstaaten:
**AT BE CH DE DK ES FR GB GR IE IT LI LU NL PT SE**

(30) Priorität: **08.05.1993 DE 4315368**

(43) Veröffentlichungstag der Anmeldung:
**17.11.1994 Patentblatt 1994/46**

(73) Patentinhaber: **HOECHST AKTIENGESELLSCHAFT**
**65929 Frankfurt am Main (DE)**

(72) Erfinder:
- **Enhsen, Alfons, Dr.**
  **D-64572 Büttelborn (DE)**
- **Glombik, Heiner, Dr.**
  **D-65719 Hofheim am Taunus (DE)**
- **Kramer, Werner, Dr.Dr.**
  **D-55130 Mainz (DE)**
- **Wess, Günter, Dr.**
  **D-63526 Erlensee (DE)**

(56) Entgegenhaltungen:
**EP-A- 0 489 423**

- **Drugs of the Future Band 17(7) S.575-593 (1992)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 624 596 B1

Printed by Jouve, 75001 PARIS (FR)

**Beschreibung**

Die Erfindung betrifft Tetrazolgallensäurederivate der Formel I

G1 - X - G2 I,

Verfahren zu ihrer Herstellung, pharmazeutische Präparate auf Basis dieser Verbindungen sowie die Verwendung der Gallensäurederivate als Arzneimittel.

Die erfindungsgemäßen Verbindungen besitzen eine hohe Affinität zum spezifischen Gallensäuretransportsystem des Dünndarms und hemmen die Gallensäureresorption in konzentrationsabhängiger und kompetitiver Weise.

Gallensäuren haben eine wichtige physiologische Funktion bei der Fettverdauung, z. B. als Cofaktoren der pankreatischen Lipasen und als natürliche Detergentien zur Solubilisierung von Fetten und fettlöslichen Vitaminen. Als Endprodukt des Cholestinstoffwechsels werden sie in der Leber synthetisiert, in der Gallenblase gespeichert und aus dieser durch Kontraktion in den Dünndarm abgegeben, wo sie ihre physiologische Wirkung entfalten. Der größte Teil der sezernierten Gallensäuren wird über den enteroheptaischen Kreislauf zurückgewonnen. Sie gelangen über die Mesenterialvenen des Dünndarms und das Pfortadersystem wieder zur Leber zurück. Bei der Rückresorption im Darm spielen sowohl aktive als auch passive Transportprozesse eine Rolle. Die Hauptmenge der Gallensäuren wird am Ende des Dünndarms, dem terminalen Ileum, durch ein spezifisches $Na^+$-abhängiges Transportsystem rückresorbiert und gelangt mit dem Mesenterialvenenblut über die Pfortader zur Leber zurück, um von den Leberzellen erneut in die Galle sezerniert zu werden. Im enterohepatischen Kreislauf treten die Gallensäuren sowohl als freie Säuren als auch in Form von Glycin- und Taurinkonjugaten in Erscheinung.

Nichtresorbierbare, unlösliche, basische, vernetzte Polymere werden seit geraumer Zeit zur Bindung von Gallensäuren verwendet und aufgrund dieser Eigenschaften therapeutisch genutzt. In der Patentanmeldung EP-A-0 489 423 beschriebene Gallensäurederivate besitzen eine hohe Affinität zum intestinalen Gallensäuretransportsystem und erlauben daher eine spezifische Hemmung des enterohepatischen Kreislaufs. Als Therapieobjekt werden alle Erkrankungen, bei denen eine Hemmung der Gallensäureresorption im Darm, insbesondere im Dünndarm, wünschenswert erscheint, angesehen. Beispielsweise werden die cholagene Diarrhoe nach Ileumresektion, oder auch erhöhte Cholesterin-Blutspiegel auf diese Weise behandelt. Im Falle des erhöhten Cholesterin-Blutspiegels kann durch den Eingriff in den enterohepatischen Kreislauf eine Senkung dieses Spiegels erreicht werden. Durch Senkung des im enterohepatischen Kreislauf befindlichen Gallensäurepools wird die entsprechende Neusynthese von Gallensäuren aus Cholesterin in der Leber erzwungen. Zur Deckung des Cholesterinbedarfs in der Leber wird auf das im Blutkreislauf befindliche LDL-Cholesterin zurückgegriffen, wobei die hepatischen LDL-Rezeptoren vermehrt zum Einsatz kommen. Die so erfolgte Beschleunigung des LDL-Katabolismus wirkt sich durch Herabsetzung des atherogenen Cholesterinanteils im Blut aus.

Die meisten natürlichen Gallensäuren besitzen in der Seitenkett am D-Ring des Steroidgerüsts eine terminale Carboxylgruppe (C-Atom 24). Die Carboxylgruppe liegt in freier Form oder konjugiert mit einer Aminosäure vor.

Die Tetrazol-5-ylgruppe stellt eine isostere Gruppe zur Carboxylfunktion dar, d. h. sie besitzt ähnliche sterische, elektronische und acide Eigenschaften wie die Carboxylfunktion selbst. Aus der medizinischen Chemie ist bekannt, daß beim Ersatz der Carboxylgruppe bestimmter Wirkstoffe durch einen Tetrazol-5-ylrest die Affinität zu Enzymen und Proteinen erhalten oder erhöht wird und somit ein besseres Wirkpotential erreicht werden kann (Drugs of the Future 1992, 17(7) 575 bis 593).

Es bestand die Aufgabe, neue Arzneimittel zu finden, die in der Lage sind, den atherogenen Cholesterinanteil im Blut herabzusetzen bzw. den enterohepatischen Kreislauf hinsichtlich erhöhter Gallensäureausscheidung und darauf folgender Senkung des Cholesterinspiegels zu beeinflussen.

Diese Aufgabe wird durch die erfindungsgemäßen Tetrazolgallensäurederivate gelöst.

Die Erfindung betrifft daher Tetrazolgallensäurederivate der Formel I

G1 - X - G2 I

in der

G1 H oder einen Rest der Formel II bedeutet,

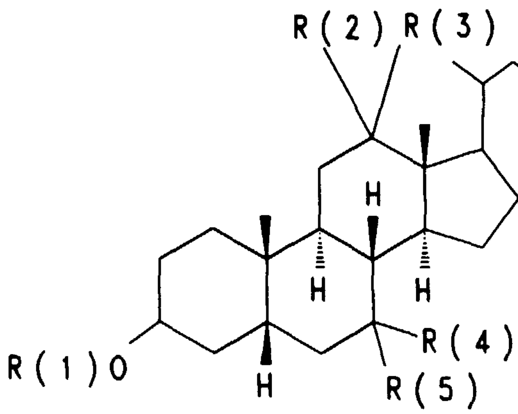

II

in der

R(1) H, einen Alkylrest oder Alkenylrest mit bis zu 10 C-Atomen, der verzweigt oder unverzweigt ist, einen Cycloalkylrest mit 3 bis 8 C-Atomen,
einen Benzylrest, der unsubstituiert oder 1- bis 3-fach substituiert ist mit F, Cl, Br, ($C_1$-$C_4$)-Alkyl oder ($C_1$-$C_4$)-Alkoxy,
einen Diphenylmethylrest, der unsubstituiert oder 1- bis 3-fach substituiert ist mit F, Cl, Br, ($C_1$-$C_4$)-Alkyl oder ($C_1$-$C_4$)-Alkoxy,
einen Triphenylmethylrest, der unsubstituiert oder 1- bis 3-fach substituiert ist mit F, Cl, Br, ($C_1$-$C_4$)-Alkyl oder ($C_1$-$C_4$)-Alkoxy,
einen $C_1$-$C_4$-Alkoxymethyl- oder einen Tetrahydroxylamylrest oder einen Rest

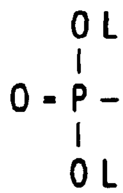 , 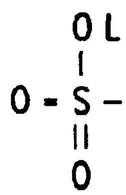 oder 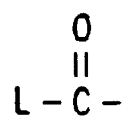

wobei L
H, einen Alkylrest oder Alkenylrest mit bis zu 10 C-Atomen, der verzweigt oder unverzweigt ist, einen Cycloalkylrest mit 3 bis 8 C-Atomen, einen Phenylrest, der unsubstituiert oder 1- bis 3-fach substituiert ist mit F, Cl, Br, ($C_1$-$C_4$)-Alkyl oder ($C_1$-$C_4$)-Alkoxy,
einen Benzylrest, der unsubstituiert oder 1- bis 3-fach substituiert ist mit F, Cl, Br, ($C_1$-$C_4$)-Alkyl oder ($C_1$-$C_4$)-Alkoxy bedeutet, darstellt,

R(2) bis R(5), wobei R(2) und R(3) bzw. R(4) und R(5) jeweils gemeinsam den Sauerstoff einer Carbonylgruppe oder einzeln und jeweils unabhängig voneinander H, -OL, -SL, -NHL, Tetrahydropyranyloxy oder $C_1$-$C_4$-Alkoxymethoxy bedeuten, wobei L die oben angegebene Bedeutung hat und

G2 einen Rest der allgemeinen Formel IV bedeutet

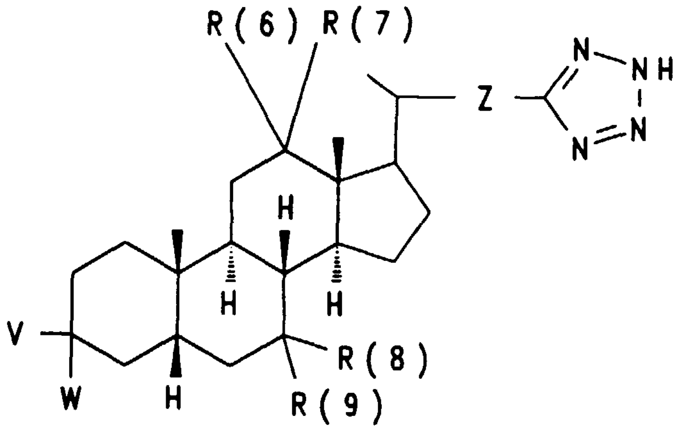

I V

wobei Z

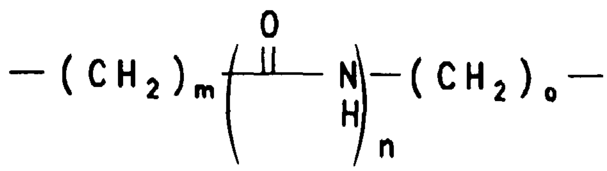

mit m = null bis 4, n = null oder 1 und o = null bis 4,

V

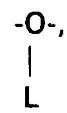

-N-, $-CH_2-$, $-CH_2CH_2-$

W H, oder falls V = $-CH_2-$, $-CH_2CH_2-$ ist, auch OH

und R(6) bis R(9) die unter R(2) bis R(5) angegebene Bedeutung haben und

X eine Brückengruppe oder eine kovalente Bindung bedeutet, wobei G1 und G2 beliebig über X verbunden sind.

Die Erfindung betrifft weiterhin Tetrazolgallensäurederivate der Formel I

G1 - X - G2 I

in der

G1 H oder einen Rest der Formel II bedeutet

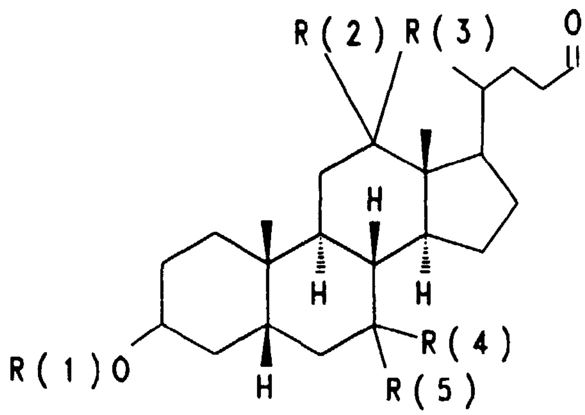

II

wobei

R(1) H, Formyl, Acetyl, Benzoyl, Methoxymethyl oder Tetrahydropyranyl,

R(2) bis R(5), wobei R(2) und R(3) bzw. R(4) und R(5) jeweils gemeinsam den Sauerstoff einer Carbonylgruppe oder einzeln und unabhängig voneinander H, OH, O-Formyl, O-Acetyl, O-Benzoyl, Methoxymethoxy oder Tetrahydropyranyloxy bedeuten,

X eine kovalente Bindung oder eine der folgenden Brückengruppen darstellt

$-NH-(CH_2)_2-$ , $-NH-(CH_2)_3-$ , $-NH-(CH_2)_6-$ , $-NH-(CH_2)_2-O-(CH_2)_2-$ ,

$$-NH-(CH_2)_3-NH-C(=O)-(CH_2)_2-C(=O)-NH-(CH_2)_2-$$

G2 einen Rest der Formel IV bedeutet

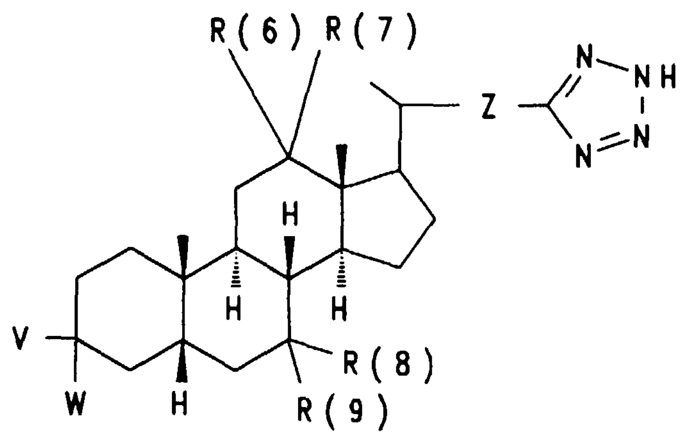

IV

wobei

V -O-,

-N-
H

W H

Z

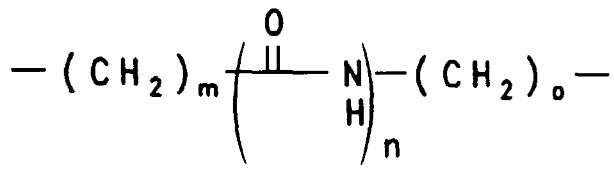

mit m = 1 bis 3, n = null oder 1 und o null, 1 oder 2 und

R(6) bis R(9) die vorstehend unter R(2) bis R(5) angegebene Bedeutung haben.

Die erfindungsgemäßen Verbindungen besitzen eine hohe Affinität zum spezifischen Gallensäuretransportsystem des Dünndarms und hemmen die Gallensäureresorption in konzentrationsabhängiger und kompetitiver Weise.

Weiterhin werden die erfindungsgemäßen Verbindungen selbst nicht resorbiert und gelangen somit nicht in den Blutkreislauf. Durch Anwendung dieses Wirkprinzips kann sehr spezifisch und effizient der enterohepatische Kreislauf der Gallensäure unterbrochen werden.

Durch Verwendung der erfindungsgemäßen Verbindungen ist es möglich, die Menge der im enterohepatischen Kreislauf befindlichen Gallensäure zu vermindern, so daß eine Senkung des Cholesterinspiegels im Serum erfolgt. Avitaminosen sind bei der Anwendung ebenso wenig zu erwarten wie die Beeinflußung der Resorption anderer Arzneimittel oder auch eine negative Wirkung auf die Darmflora. Weiterhin werden die von den Polymeren bekannten Nebenwirkungen (Obstipation, Steratorrhoe) nicht beobachtet, d. h. die Fettverdauung wird nicht negativ beeinflußt. Wegen der hohen Affinität zum spezifischen Gallensäuretransportsystem des Dünndarms kommt man mit geringen Tagesdosen aus, so daß die Akzeptanz solcher Arzneimittel bei Arzt und Patient sehr hoch sein wird.

Die Erfindung betrifft ferner ein Verfahren zur Herstellung von Verbindungen der Formel I, das dadurch gekennzeichnet ist, daß man

a) im Falle von X = Einfachbindung geeignete reaktionsfähige Formen von G1 und G2 nach im Prinzip bekannten Verfahren miteinander zur Reaktion bringt oder

b) im Falle von X = Brückengruppe

α) reaktionsfähige Formen von G1-X mit G2 bzw.
β) reaktionsfähige Formen von G2-X mit G1

nach im Prinzip bekannten Verfahren miteinander zur Reaktion bringt

a) X = Einfachbindung

Die Gallensäuren G1 werden entweder in freier Form oder in geschützter Form eingesetzt. Nach der Verknüpfung mit G2, die ebenfalls in freier oder geschützter Form vorliegt, erfolgt gegebenenfalls die Abspaltung der Schutzgruppen. Als Schutzgruppen für die Alkoholgruppen eignen sich zweckmäßigerweise Formyl, Acetyl oder Tetrahydropyranyl. Die Tetrazolylfunktion ist entweder in G2 bereits vor der Verknüpfung vorhanden oder wird nach der Verknüpfung mit G1 über eine Carboxylgruppe von G2 eingeführt.

Beispielsweise reagiert Gallensäure bevorzugt an Position 3, aber auch an Position 7 mit aktivierten Formen von Carbonsäuren, wie Säurechloriden oder gemischten Anhydriden unter Zusatz von Basen wie Trialkylamin, Pyridin, aber auch NaOH bei Zimmertemperatur in geeigneten Lösungsmitteln wie Tetrahydrofuran, Methylenchlorid oder Essigester, aber auch Dimethylformamid (DMF) oder Dimethoxyethan (DME).

Die verschienen Isomeren können z. B. chromatographisch getrennt werden.

Durch die Verwendung von geeigneten Schutzgruppen läßt sich die Reaktion selektiv durchführen.

Analog lassen sich entsprechende Aminogallensäuren in entsprechende Amide überführen. Auch hier kann die Reaktion entweder mit geschützten oder freien Gallensäuren durchgeführt werden.

Analog lassen sich weitere erfindungsgemäße Verbindungen nach bekannten Standardverfahren verknüpfen.

b) X = Brückengruppe

Die unter a) angegebenen Verfahren werden auch angewendet, um die Verknüpfung G1-X mit G2 bzw. G1 mit X-G2 durchzuführen.
Zweckmäßigerweise setzt man auch hier den Gallensäureteil entweder geschützt oder ungeschützt ein.
Ein bevorzugtes Herstellungsverfahren besteht darin, reaktionsfähige Formen von G1 mit reaktionsfähigen Formel X-G2 umzusetzen.
Gegenenfalls schließen sich nach der Verknüpfung die Abspaltung von Schutzgruppen und die Umwandlung in ein Tetrazolderivat an.
Die Herstellung von Tetrazolyl-Gallensäurebausteinen oder Tetrazolylgallensäuren ist in den folgenden Formelschemata beschrieben.

Schema 1

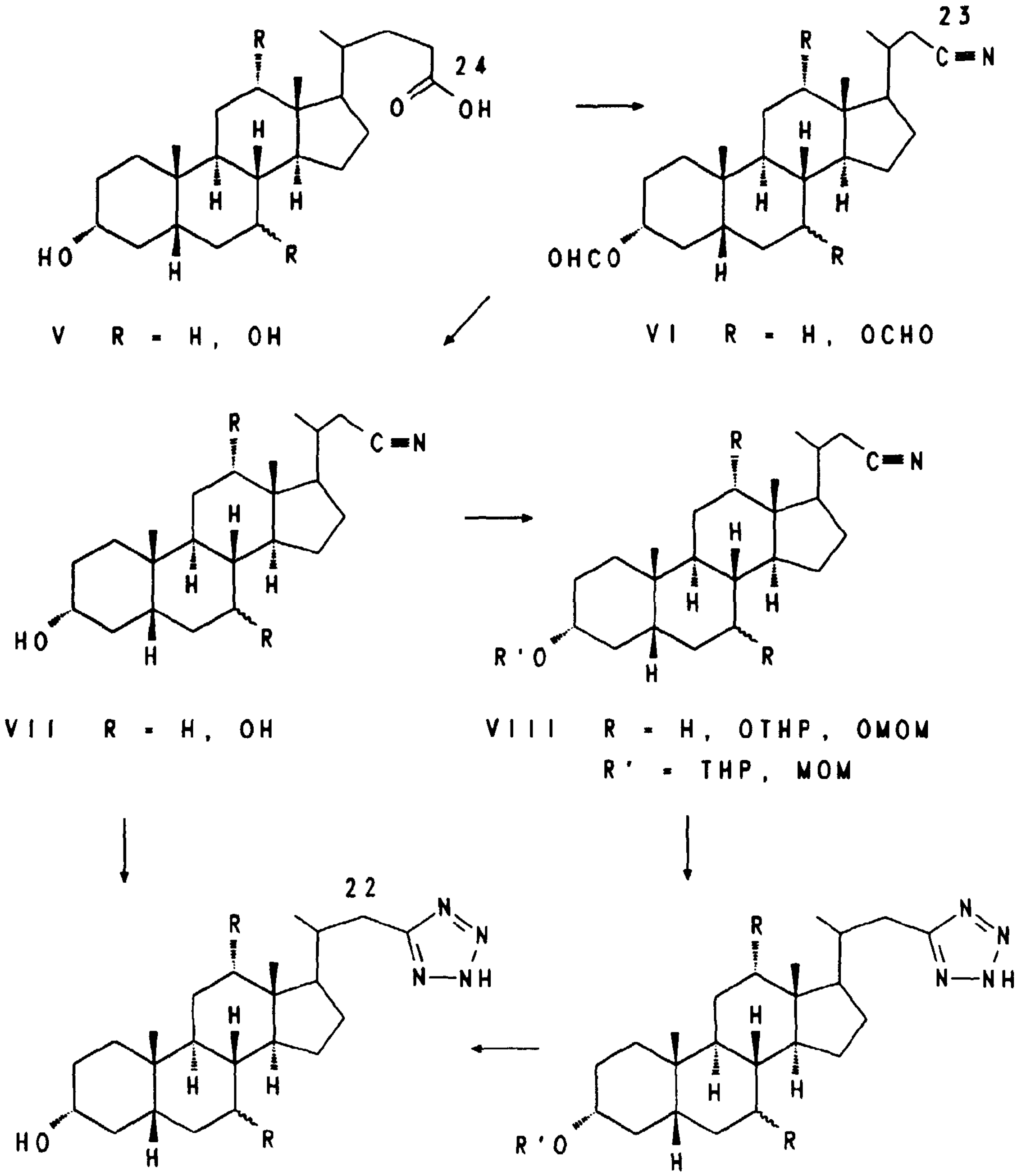

V R = H, OH

VI R = H, OCHO

VII R = H, OH

VIII R = H, OTHP, OMOM
R' = THP, MOM

X R = H, OH

IX R = H, OTHP, OMOM
R' = THP, MOM

THP = Tetrahydropyranyl, MOM = Methoxymethyl

C-22-Tetrazolyl-Cholansäurederivate X sind zugänglich, indem zunächst die Seitenkette einer C-24-Cholansäure V

um ein C-Atom zum Nitril VI verkürzt wird (J. Lip. Res. 29, 1387, 1988). Durch Verseifung unter milden Bedingungen werden die Schutzgruppen abgespalten und es werden Verbindugnen vom Typ VII erhalten. Diese Nitrile VII können nun direkt mit Trialkylzinnaziden in einem geeigneten Lösungsmittel, wie z. B. Toluol, bei erhöhten Temperaturen zu den Tetrazolderivaten X umgesetzt werden. Es kann jedoch vorteilhaft sein, die freien OH-Gruppen vor dem Tetrazolbildungsschritt zu schützen, z. B. mit THP- oder MOM-Schutzgruppen zu Verbindungen VIII. Die Reaktion zum geschützten Tetrazolderivat IX erfolgt unter den oben genannten Bedingungen. Abspaltung der Schutzgruppen führt wiederum zu Verbindungen des Typs X.

## Schema 2

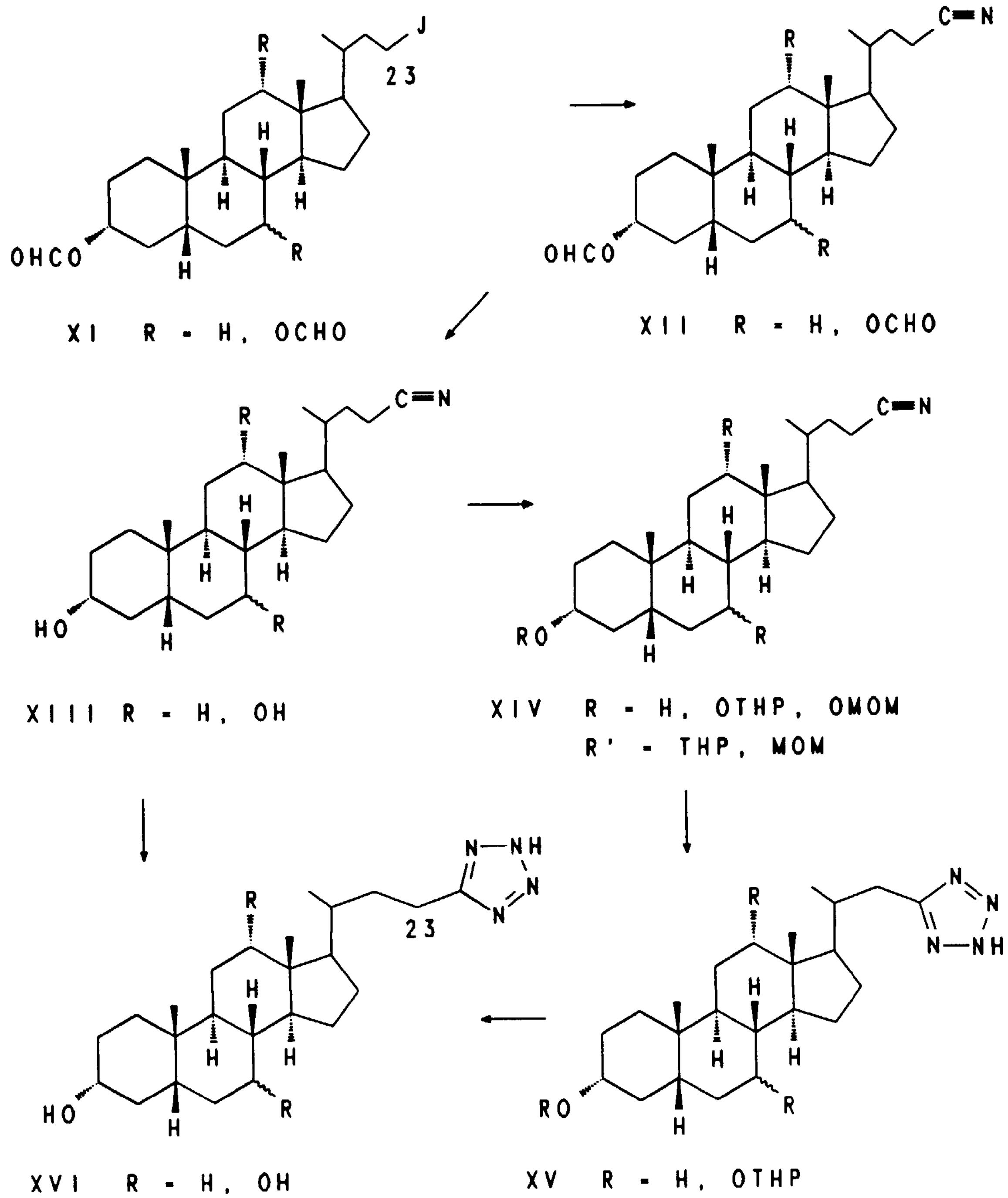

XI R = H, OCHO

XII R = H, OCHO

XIII R = H, OH

XIV R = H, OTHP, OMOM
R' = THP, MOM

XVI R = H, OH

XV R = H, OTHP

Zur Herstellung von C-23-Tetrazolylcholansäuren XVI werden beispielsweise Jodverbindungen des Typs XI durch nu-

kleophile Substitution mit Alkalicyaniden in Nitrile XII überführt. Nach Abspaltung der Schutzgruppen lassen sich die Nitrile XIII entweder direkt zu den Tetrazolverbindungen XVI umsetzen oder es kann auch die Möglichkeit über die geschützten Derivate XIV und XV genutzt werden.

## Schema 3

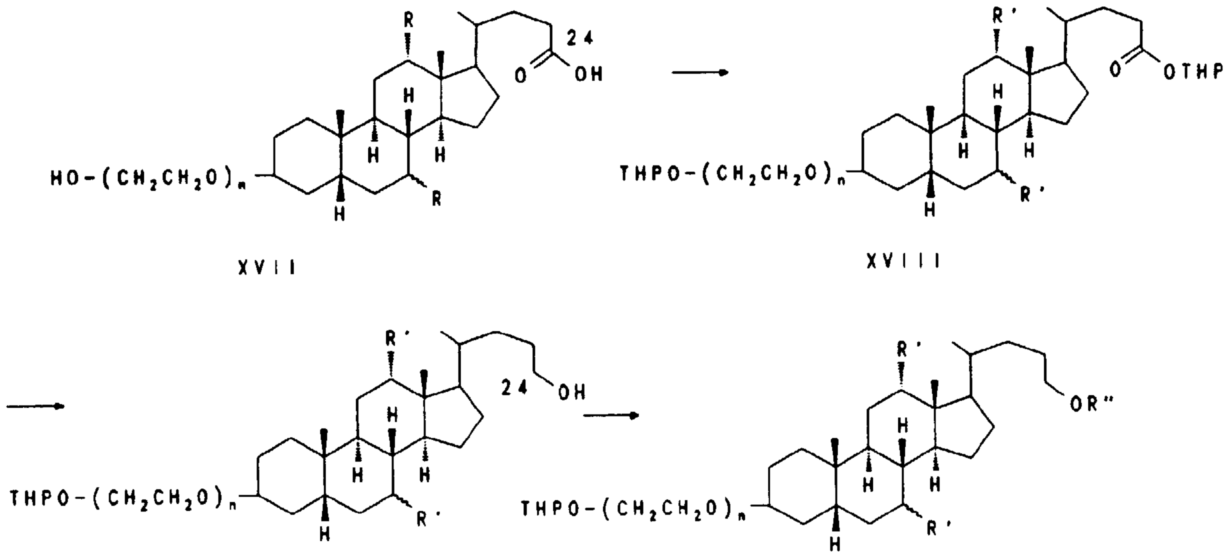

XVII

XVIII

XIX

XX

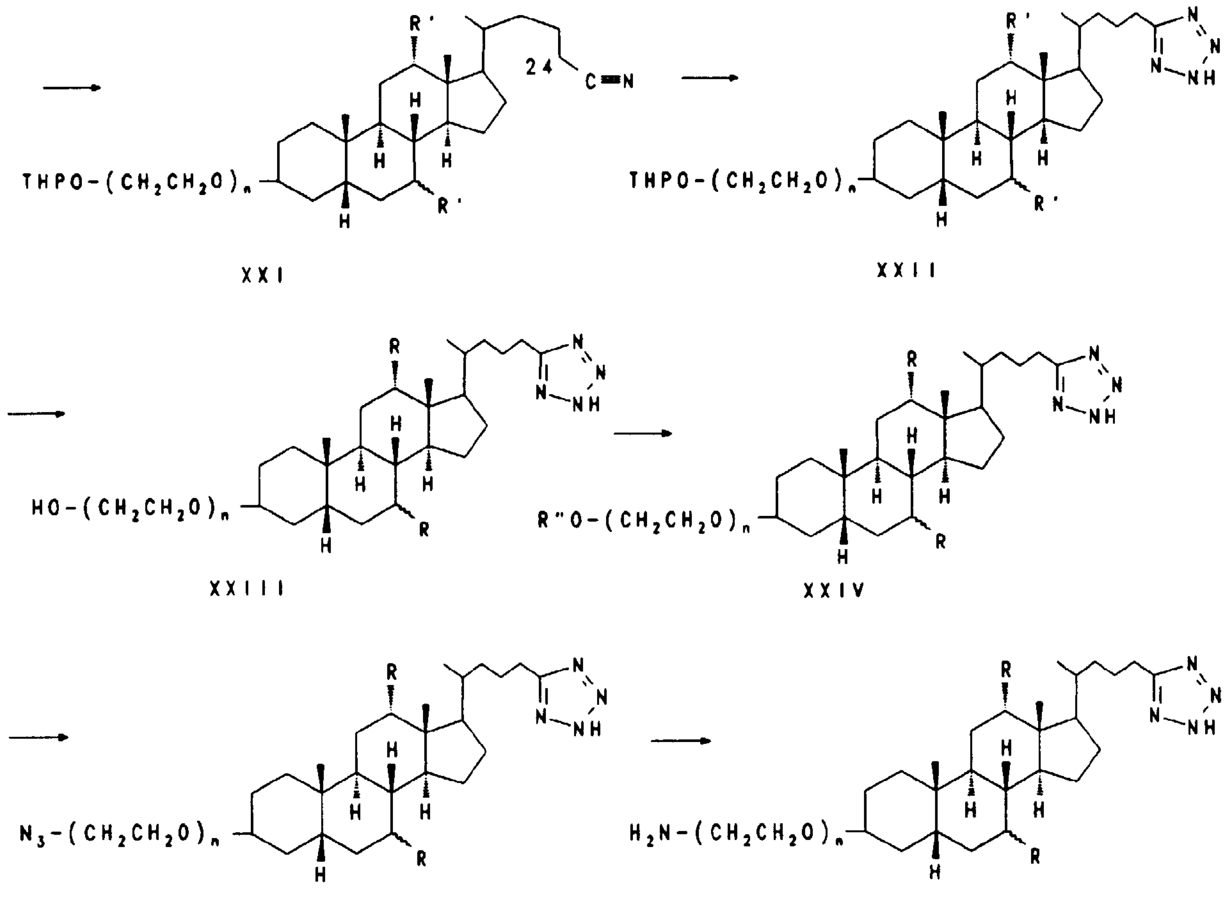

XXI XXII

XXIII XXIV

XXV XXVI

R = H, OH; R' = H, OTHP; R" = Mesyl, Tosyl; n = 0, 1, 2

C-24-Tetrazolderivate werden erhalten, indem Verbindungen des Typs XVII an den Hydroxygruppen und an der Säurefunktion mit Schutzgruppen versehen werden. Die entstandenen Esterfunktionen von Verbindungen des Typs XVIII werden zu primären Hydroxygruppen reduziert (XIX). Die Hydroxygruppe wird durch einen Methan- oder Toluolsulfonylrest aktiviert (XX) und mit einem Alkalicyamid substituiert; dadurch werden Verbindungen des Typs XXI erhalten. Die Reaktion von Nitril- zu Tetrazolylverbindungen (XXII) und die anschließende Abspaltung der Schutzgruppen zu XXIII erfolgt nach den oben beschriebenen Verfahren. Aus der freien Hydroxyfunktion in Position 3 oder im eingeführten Bindeglied X kann analog zu bereits beschriebenen Verfahren (EP-A-O 489 423) über Verbindungen der Formeln XXIV und XXV eine Aminofunktion hergestellt werden und es werden Verbindungen des Typs XXVI erhalten.

## Schema 4

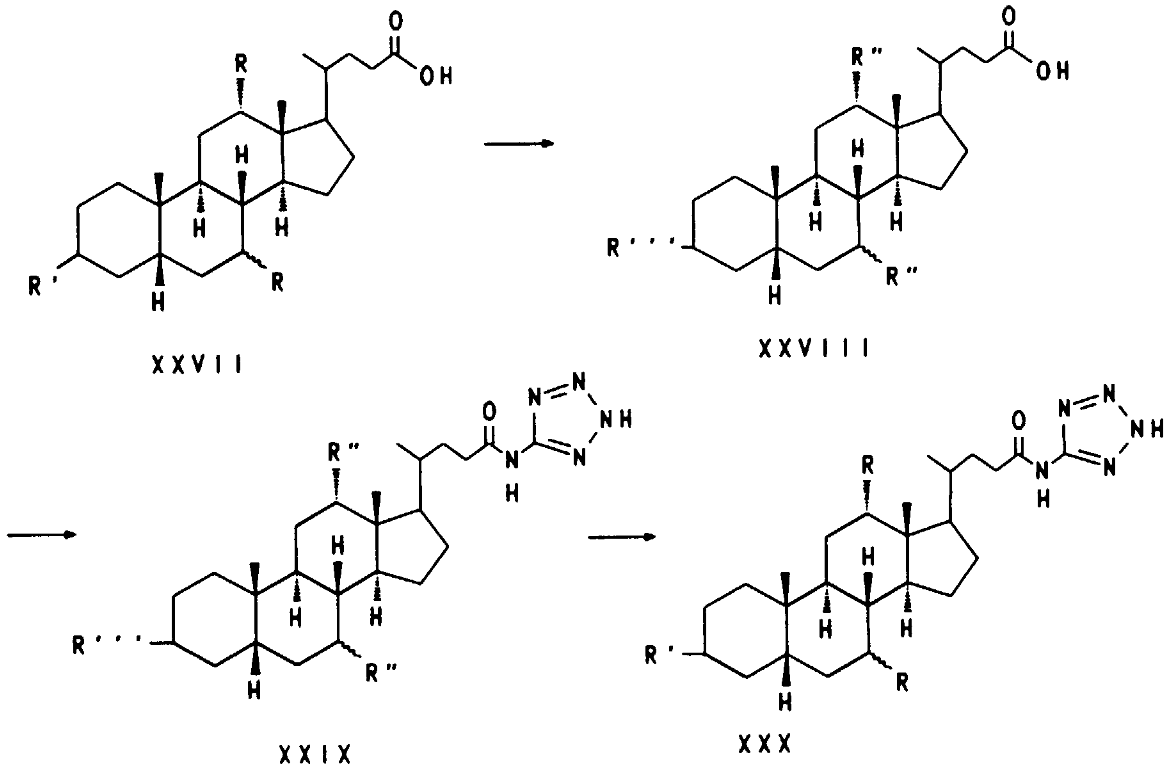

XXVII

XXVIII

XXIX

XXX

R = H, OH

R' = OH oder

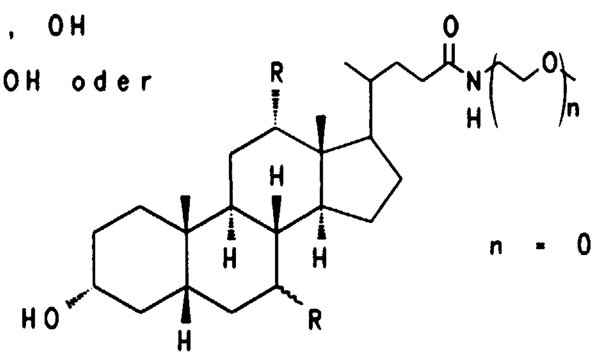

n = 0, 1, 2

R" = H, OCHO, $OCOCH_3$

R''' = OCHO, $OCOCH_3$ oder

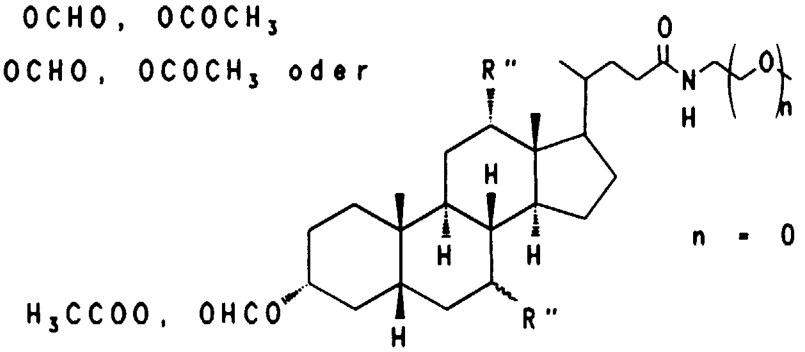

n = 0, 1, 2

Aus natürlichen Gallensäuren oder auch aus modifizierten Gallensäuren, z. B. aus dimeren Derivaten, lassen sich Tetrazolderivate darstellen, indem die freie Carboxylgruppe nach Aktivierung mit 5-Aminotetrazol zur Reaktion gebracht wird. Bei Verwendung der üblichen Peptid-Kupplungsreagenzien sind die Ausbeuten bei dieser Reaktion gering. Daher ist es zweckmäßig die freien Hydroxyfunktionen des Gallensäurederivates XXVII zu schützen, z. B. mit Formyl- oder Acetylschutzgruppen. Aus den geschützten Verbindungen XXVIII wird mit Phosphorpentachlorid oder Thionylchlorid, z. B. in THF, das Säurechlorid erzeugt. Durch Reaktion des Säurechlorids mit trockenem 5-Aminotetrazol erhält man Verbindungen des Typs XXIX, aus denen durch einfache Abspaltung der Schutzgruppen die Endprodukte XXX erhalten werden.

Die Verbindungen besitzen wertvolle pharmakologische Eigenschaften und eignen sich daher insbesondere als Hypolipidämika.

Die Erfindung betrifft daher auch Arzneimittel auf Basis der Verbindungen der Formel (I) sowie die Verwendung der Verbindungen als Arzneimittel, insbesondere zur Senkung des Cholesterinspiegels.

Die biologische Prüfung der erfindungsgemäßen Verbindungen erfolgte durch Ermittlung der Hemmung der [3H]-Taurocholataufnahme in Bürstensaummembranvesikel des Ileums von Kaninchen. Der Hemmtest wurde wie folgt durchgeführt:

1. Präparation von Bürstensaummembranvesikeln aus dem Ileum von Kaninchen

Die Präparation von Bürstensaummembranvisikeln aus den Darmzellen des Dünndarm erfolgte mit der sogenannten $Mg^{2+}$-Präzipitationsmethode. Männliche Neuseeland-Kaninchen (2 bis 2,5 kg Körpergewicht) wurden durch intravenöse Injektion von 0,5 ml einer wäßrigen Lösung von 2,5 mg Tetracain HCl, 100 T 61$^R$ und 25 mg Mebezoniumjodid getötet. Der Dünndarm wurde entnommen und mit eiskalter physiologischer Kochsalzlösung gespült. Die terminalen 7/10 des Dünndarms (gemessen in oral rectaler Richtung, d. h. das terminale Ileum, welches das aktive $Na^+$-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethylsulfonylfluorid/1 mg/l Trypsin Inhibitor v. Sojabohnen (32 (U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, BRD) 3 Minuten bei 75 % max. Leistung unter Eiskühlung homogenisiert. Nach Zugabe von 3 ml 1 M $MgCl_2$-Lösung (Endkonzentration 10 mM) ließ man exakt 1 Minute bei 0°C stehen. Durch Zugabe von $Mg^{2+}$ aggregieren die Zellmembranen und präzipitieren mit Ausnahme der Bürstensaummembranen. Nach einer 15-minütigen Zentrifugation bei 3000 x g (5000 rpm, SS-34-Rotor) wird der Niederschlag verworfen und der Überstand, der die Bürstensaummembranen enthält, 30 Minuten bei 267000 x g (15000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M $MgCl_2$-Lösung und 15-minütiger Inkubationszeit bei 0°C wurde erneut 15 Minuten bei 3000 x g zentrifugiert. Der Überstand wurde anschließend nochmals 30 Minuten bei 46000 x g (15000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

2. Hemmung der $Na^+$-abhängigen [3H]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [$^3$H(G)]-Taurocholat (spezifische Aktivität: 2,1 Ci/mMol), /0,5 µl 10 mM Taurocholat/8,75 µl Natrium-Transport-Puffer (10 mM Tris/Hepes, (pH 7,4)/100 mM Mannit/100 mM NaCl) (Na-T-P) bzw. 8,75 pl Kalium-Transport-Puffer (10 mM Tris/Hepes (pH 7,4)/ 100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm Ø, Millipore, Eschborn, BRD) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes, (pH 7,4)/150 mM KCl) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher &

Schuell, Dassell, BRD) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, BRD) aufgelöst und die Radioaktivität durch Flüssigkeits-Szintillationsmessung in einem Meßgerät TriCarb 2500 (Canberra Packard GmbH, Frankfurt, BRD) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den $Na^+$-abhängigen Transportanteil. Als $IC_{50}$ $Na^+$ wurde diejenige Konzentration an Inhibitor bezeichnet, bei der der $Na^+$-abhängige Transportanteil um 50 % - bezogen auf die Kontrolle - gehemmt war.

Die pharmakologischen Daten umfassen eine Testserie, in der die Interaktion der erfindungsgemößen Verbindungen mit dem intestinalen Gallensäuretransportsystem im terminalen Dünndarm untersucht wurde. Die Ergebnisse sind in Tabelle 1 zusammengefaßt.

Die Erfindung betrifft weiterhin die Verwendung der erfindungsgemäßen Verbindungen zur Herstellung eines Heilmittels.

Hierzu werden die Verbindungen der Formel I gelöst oder suspendiert in pharmakologisch unbedenklichen organischen Lösungsmitteln, wie ein- oder mehrwertigen Alkoholen, wie z. B. Ethanol oder Glycerin, in Triacetin, Ölen, z. B. Sonnenblumenöl, Lebertran, Ethern, wie z. B. Diethylenglykoldimethylether, oder auch Polyethern, z. B. Polyethylenglykol, oder auch in Gegenwart anderer pharmakologisch unbedenklicher Polymerträger, wie z. B. Polyvinylpyrrolidon, oder anderen pharmazeutisch annehmbaren Zusatzstoffen wie Stärke, Cyclodextrin oder Polysacchariden. Ferner können die erfindungsgemäßen Verbindungen in Kombination mit anderen Arzneistoffe, gegeben werden.

Die Verbindungen der Formel I werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 5000 mg, vorzugsweise jedoch in Dosisbereich 10 bis 1000 mg.

Beispiel 1

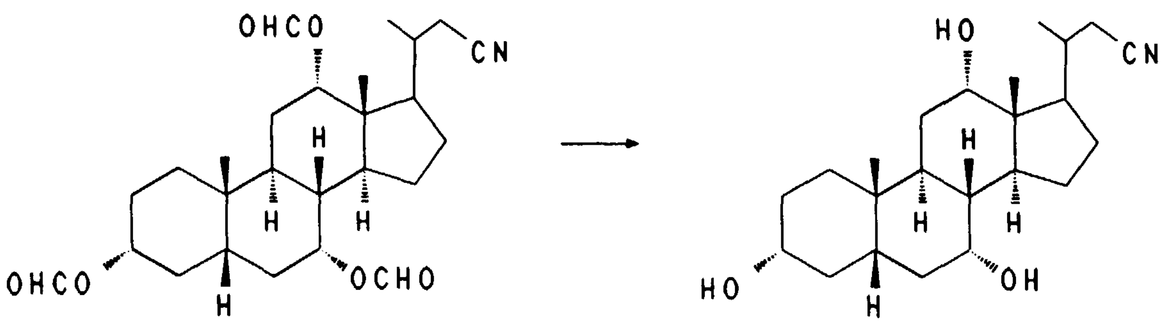

5,0 g (10,9 mmol) Triformylverbindung (J. Lip. Res. 29, 1387, 1988) werden in 100 ml 1M NaOMe/MeOH-Lösung 2 Stunden bei Zimmertemperatur entschützt. Zur Aufarbeitung wird Wasser zugegeben und das Methanol abgezogen. Es wird dreimal mit $CH_2Cl_2$ extrahiert, über $MgSO_4$ getrocknet und eingeengt. Es werden 3,9 g (96 %) des ungeschützten Nitrils erhalten.

MS (FAB, 3-NBA/LiCl) $C_{33}H_{37}NO_3$ (375) 383 ($M+Li^+$)

Beispiel 2

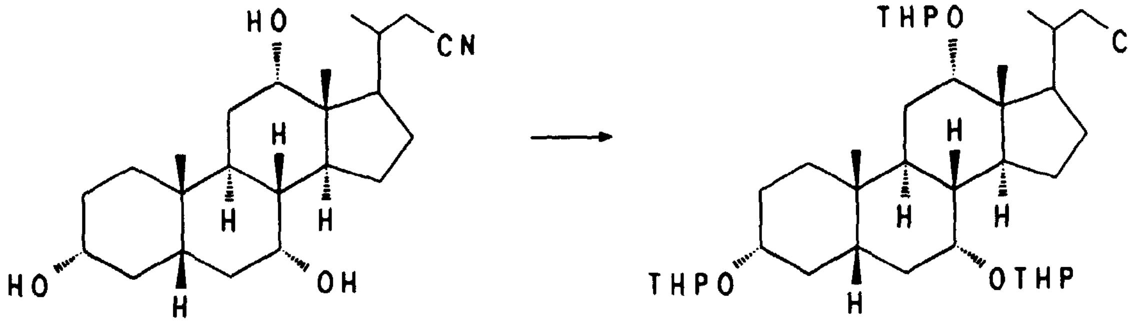

3,8 g (10,1 mmol) Trihydroxyverbindung (Beispiel 1) werden in 40 ml $CH_2Cl_2$ gelöst, bei 0°C mit 20 ml Dihydropyran und 300 mg Pyridinium-(toluol-4-sulfonat) versetzt und anschließend 2 Tage bei Zimmertemperatur gerührt. Das Reaktionsgemisch wird eingeengt, und der Rückstand wird über Kieselgel chromatographiert (Cyclohexan/Essigester 2: 1). Es werden 5,7 g (90 %) Produkt erhalten.
MS (FAB, 3-NBA/LiCl) $C_{33}H_{53}NO_5$ 8543) 550 ($M+Li^+$)

Beispiel 3

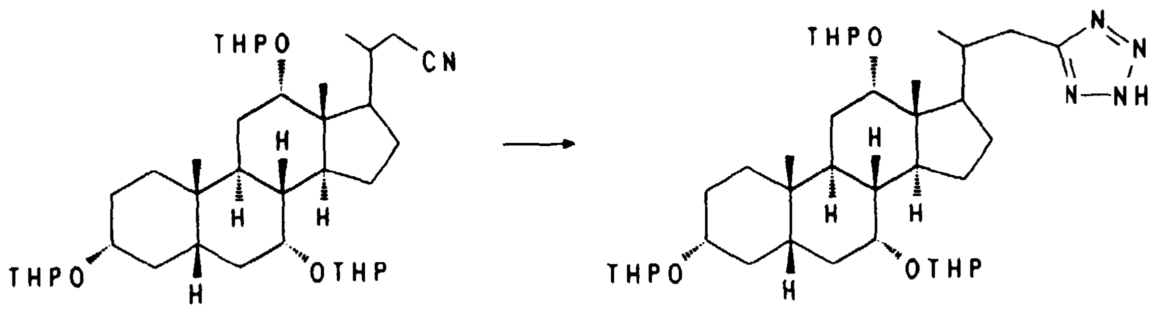

2,0 g (3,19 mmol) Nitril (Beispiel 2) und 2,1 g (6,34 mmol) Tributylzinnazid werden in Toluol 6 Tage unter Rückfluß erhitzt. Nach 3 Tagen werden nochmals 2,1 g Tributylzinnazid zugegeben. Das Reaktionsgemisch wird eingeengt. Nach Chromatographie des Rückstands (Kieselgel, Essigester/MeOH 95:5) werden 1,7 g (79 %) Tetrazolderivat erhalten.
MS (FAB), 3-NBA/LiCl) $C_{38}H_{62}N_4O_6$ (670) 677 ($M+Li^+$)

Beispiel 4

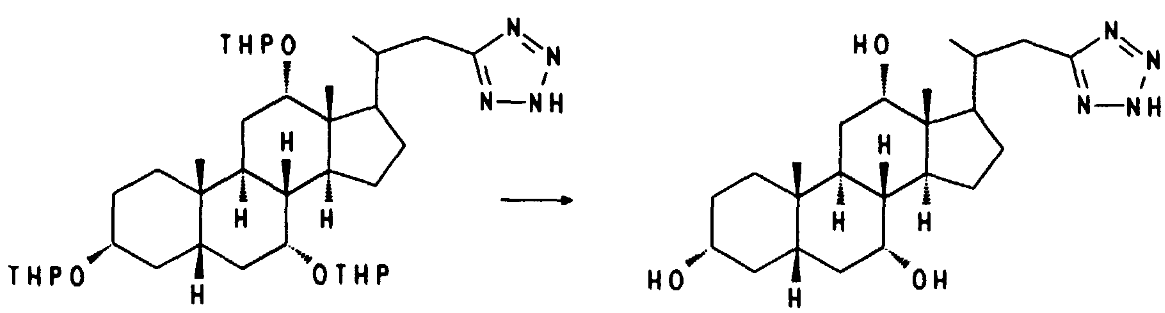

1,6 g (2,39 mmol) THP-geschützte Verbindung (Beispiel 3), 200 mg Pyridinium-p-toluolsulfonat und 1,6 ml Essigsäure werden in 30 ml Methanol 12 Stunden unter Rückfluß erhitzt. Nach dem Abkühlen wird eingeengt und der Rückstand über Kieselgel ($CH_2Cl_2$/MeOH 9:1) chromatographiert. Ausbeute: 720 mg (87 %)

MS (FAB, 3-NBA/LiCl) $C_{23}H_{38}N_4O_3$ (418) 425 (M+Li+)

Beispiel 5

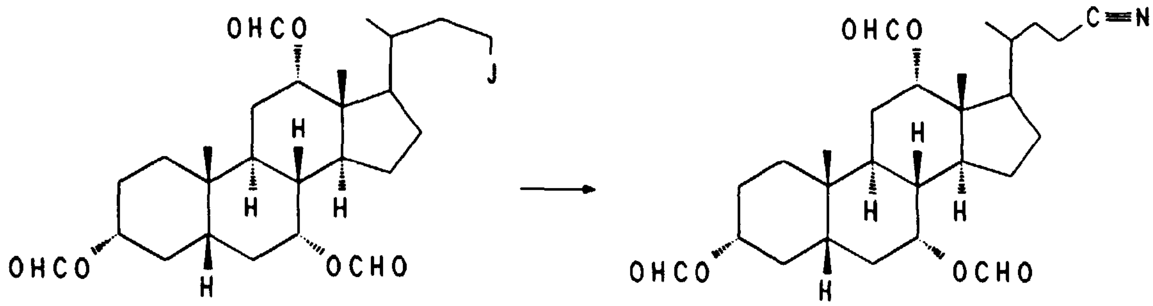

21 g (36,6 mmol) Jodverbindung (Tetrahedron, 45 (17), 5423, 1989) und 3,6 g Natriumcyanid werden in 300 ml DMSO 1,5 Stunden bei 50°C gerührt. Das Reaktionsgemisch wird auf Eiswasser gegossen und mit Essigester extrahiert. Die organische Phase wird getrocknet ($MgSO_4$) und eingeengt.
Chromatographie (Cyclohexan/Essigester) liefert 15 g (87 %) Nitril.
MS (FAB, 3-NBA/LiCl) $C_{27}H_{39}NO_6$ (473) 480 (M+Li+)

Beispiel 6

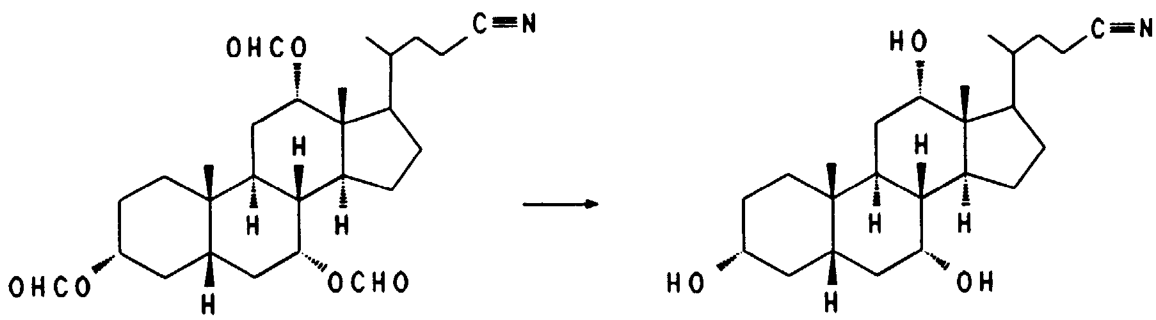

Die formylgeschützte Verbindung (Beispiel 5) wird nach dem für Beispiel 1 beschriebenen Verfahren entschützt.
MS (FAB, 3-NBA/LiCl) $C_{24}H_{39}NO_3$ (389) 396 (M+Li+)
Die Beispiele 7, 8 und 9 wurden nach dem für die Beispiele 2, 3 und 4 beschriebenen Verfahren hergestellt.

Beispiel 7

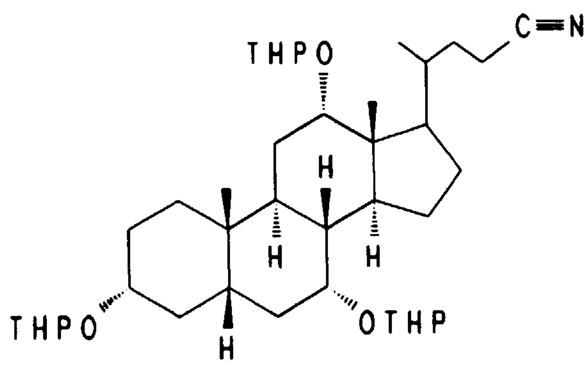

MS (FAB, 3-NBA/LiCl) $C_{39}H_{63}NO_6$ (641) 648 (M+Li+)

Beispiel 8

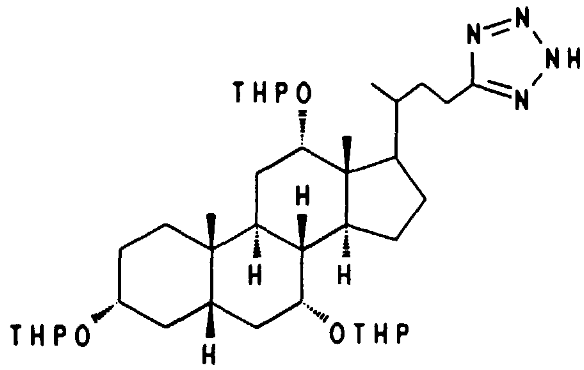

MS (FAB, 3-NBA/LiCl) $C_{39}H_{64}N_4O_6$ (684,5) 691,6 (M+Li+)

Beispiel 9

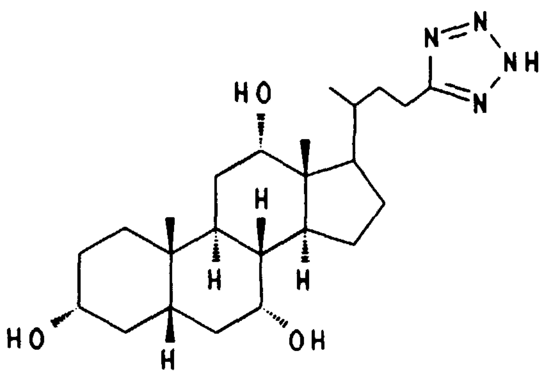

MS (FAB, 3-NBA/LiCl) $C_{24}H_{40}N_4O_3$ (432) 439 (M+Li+)

Die Verbindung des Beispieles 9 kann auch direkt aus Beispiel 6 und Tributylzinnazid (siehe Beispiel 3) erhalten werden. Das Rohprodukt wird zur Reinigung zweimal chromatographiert (1. Essigester/MeOH 9:1; 2. $CH_2Cl_2$/MeOH/Essigsäure 9:1:0,1). Die Ausbeute beträgt 83 %.

Beispiel 10

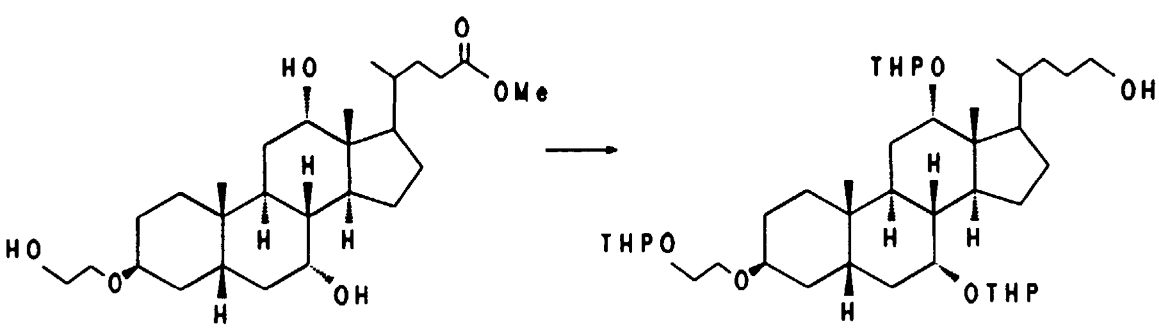

100 g (215 mmol) Trihydroxyverbindung (EP-A-0 489 423) werden nach Beispiel 2 mit Tetrahydropyranylgruppen geschützt. Das erhaltene Rohprodukt wird ohne weitere Reinigung umgesetzt. Gelöst in 250 ml Ether wird es langsam

bei 0°C zu einer Suspension von 15 g $LiAlH_4$ in 500 ml Ether zugetropft. Nach 2 Stunden bei 0 bis 5°C wird vorsichtig mit Wasser versetzt und anschließend mehrfach mit Ether extrahiert. Die organische Phase wird getrocknet ($MgSO_4$) und eingeengt. Es werden 100 g (67 %) Produkt erhalten.

Beispiel 11

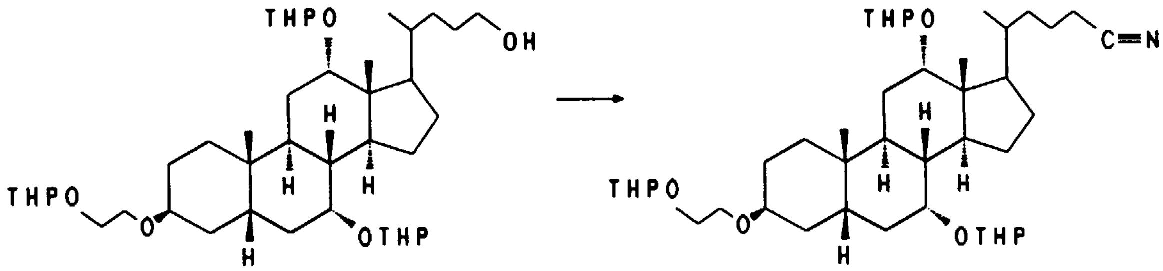

20 g (29,0 mmol) des Alkohols (Beispiel 10) werden in 150 ml Pyridin gelöst. Bei 0°C werden langsam 2,45 ml (31,0 mmol) Methansulfonsäurechlorid zugetropft und anschließend wird 2 Stunden bei Zimmertemperatur gerührt. Zur Aufarbeitung wird auf Eiswasser gegossen und mit Essigester extrahiert. Die organische Phase wird getrocknet ($MgSO_4$) und eingeengt. Das Rohprodukt wird in 200 ml DMSO gelöst, mit 3,0 gr NaCN versetzt und 1 Stunde bei 50°C gerührt. Es wird auf Eiswasser gegossen und mit Essigester extrahiert. Nach dem Trocknen der organischen Phase wird eingeengt und chromatographiert (Cyclohexan/Essigester 7:3). Es werden 15 g (77 %)( Nitril erhalten. MS (FAB, 3-NBA/LiCl) $C_{42}H_{69}NO_7$ (699) 706 ($M+Li^+$)

Beispiel 12

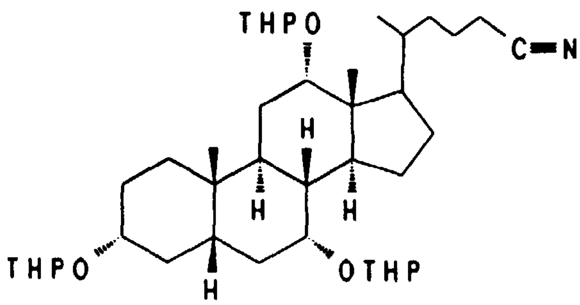

Beispiel 12 wurde nach dem für Beispiel 11 beschriebenen Verfahren erhalten.

Die Beispiel 13 bis 16 werden nach den für die Beispiele 3 und 4 beschriebenen Verfahren hergestellt.

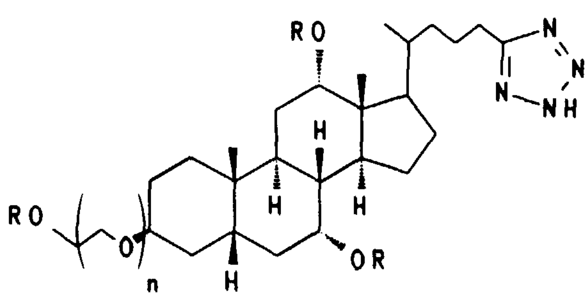

| Beispiel | R | n | MS (FAB, 3-NBA/LiCl) |
|---|---|---|---|
| 13 | THP | 0 | $C_{40}H_{66}N_4O_6$ (698,5) 705,7 ($M+Li^+$) |

(fortgesetzt)

| Beispiel | R | n | MS (FAB, 3-NBA/LiCl) |
|---|---|---|---|
| 14 | H | 0 | $C_{25}H_{42}N_4O_3$ (446) 453 ($M+Li^+$) |
| 15 | THP | 1 | $C_{42}H_{70}N_4O_7$ (742,5) 749,4 ($M+Li^+$) |
| 16 | H | 1 | $C_{27}H_{46}N_4O_6$ (490) 497 ($M+Li^+$) |

Beispiel 17

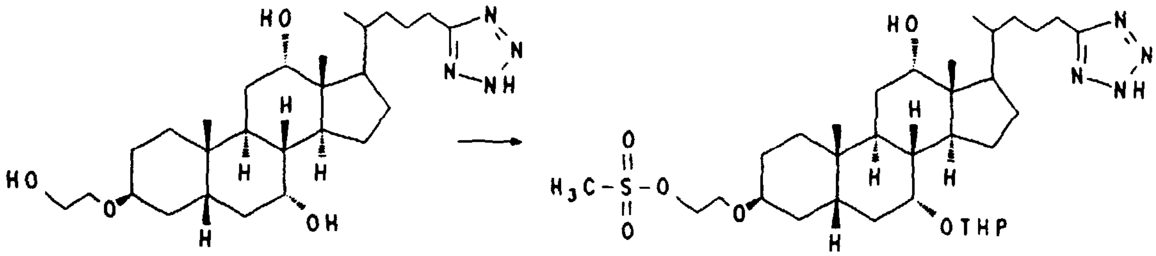

15 g (30,6 mmol) des Alkohols aus Beispiel 16 werden in 200 ml $CH_2Cl_2$ gelöst, mit 100 ml Pyridin versetzt und bei -20 bis -10°C 2,5 Stunden mit 5 ml Methansulfonsäurechlorid zur Reaktion gebracht. Die noch kalte Reaktionslösung wird auf Eiswasser gegossen, mit Essigester extrahiert, die organische Phase getrocknet ($MgSO_4$) und eingeengt. Das Rohprodukt wird über eine kurze Kieselgelsäule gereinigt ($CHCl_3$/MeOH 93:7). Es werden 16 g (92 %) Produkt erhalten.

MS (FAB, 3-NBA/LiCl) $C_{28}H_{48}N_4O_6S$(568) 575 ($M+Li^+$)

Beispiel 18

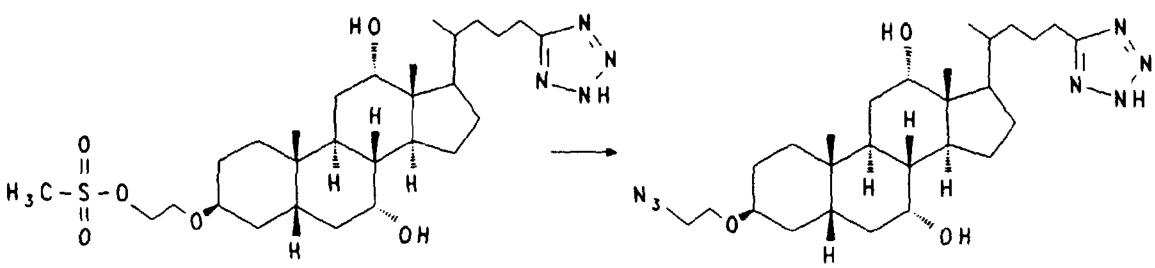

2,3 g 84,05 mmol) Mesylverbindung (Beispiel 17) und 290 mg Natriumazid werden in 30 ml DMF 2 Stunden bei 100°C gerührt. Nach dem Abkühlen wird das Reaktiongemisch eingeengt und der Rückstand über Kieselgel chromatographiert ($CH_2Cl_2$/MeOH 85/15). Die Ausbeute beträgt 1,0 g (49 %).

MS (FAB, 3-NBA/LiCl) $C_{27}H_{45}N_7O_3$ (515) 522 ($M+Li^+$)

Beispiel 19

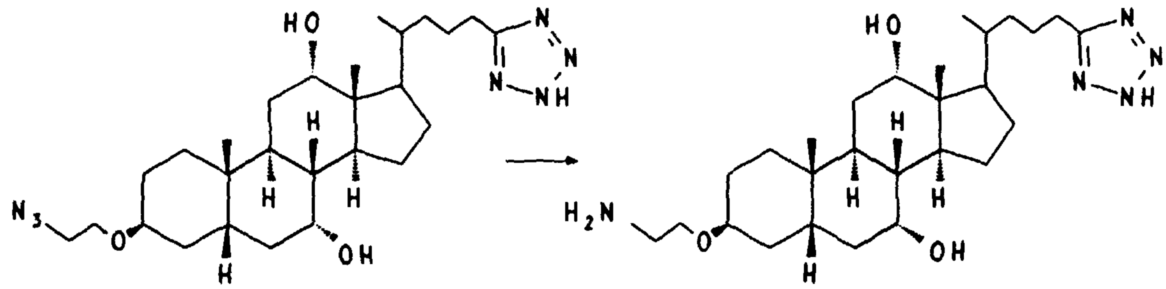

1,0 g (1,94 mmol) Azidoverbindung werden in 30 ml Methanol/1,5 ml Wasser gelöst und in Gegenwart von 50 mg Pd schwarz mit $H_2$ hydriert. Es wird vom Katalysator abfiltriert und eingeengt. Der Rückstand wird über Kieselgel chromatographiert ($CH_2Cl_2$/MeOH/$NEt_3$ 8:2:1) und es werden 550 mg (58 %) Amin erhalten.
MS (FAB, 3-NBA/LiCl) $C_{27}H_{47}N_5O_3$ (489) 496 ($M+Li^+$)

Beispiel 20

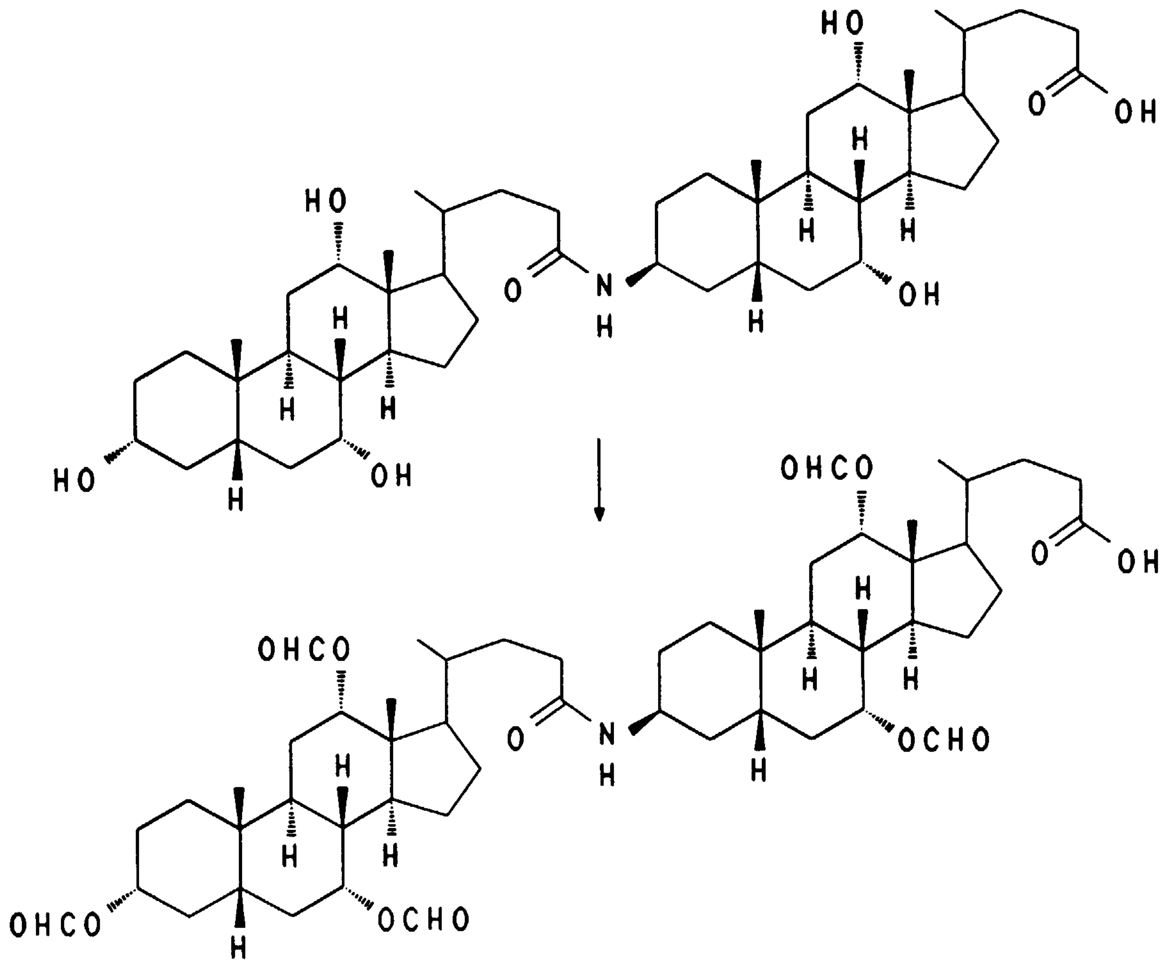

3,0 g (3,76 mmol) der ungeschützten Verbindung (EP-A-0 489 423) werden in 30 ml Ameisensäure gelöst, mit 0,2 ml Perchlorsäure versetzt und 2 Stunden bei 50°C gerührt. Anschließend werden bei Zimmertemperatur 30 ml Acetanhydrid zugetropft, und es wird noch 30 Minuten gerührt. Man gießt auf Eiswasser und extrahiert mit Essigester. Die organische Phase wird getrocknet, eingeengt und der Rückstand chromatographiert. Es werden 1,93 g (55 %) formylgeschützte Verbindung erhalten.

MS (FAB, 3-NBA) $C_{53}H_{79}NO_{13}$ (938) 939 (M+H+)

Beispiel 21

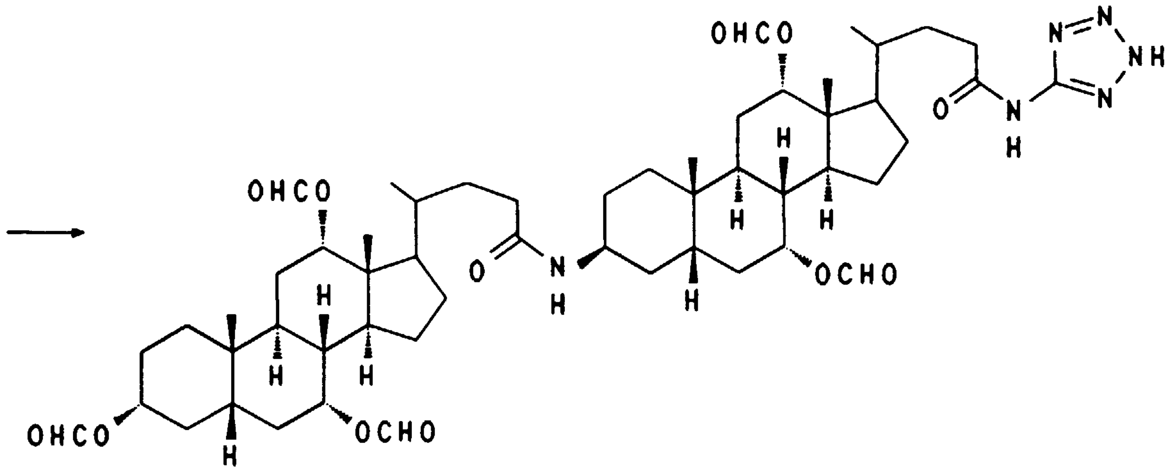

0,5 g (0,53 mmol) Beispiel 20 werden in 30 ml trockenem THF gelöst, mit 130 mg (0,62 mmol) $PCl_5$ versetzt und 30 Minuten bei Zimmertemperatur gerührt. Zur Reaktionslösung gibt man eine Lösung von 200 mg (2,35 mmol) wasserfreiem 5-Aminotetrazol in 10 ml DMF. Nach weiteren 3 Stunden bei Zimmertemperatur wird eingeengt und der Rückstand chromatographiert ($CHCl_3$/MeOH 9:1). Es werden 400 mg (75 %) Tetrazolverbindung erhalten.
MS (FAB, 3-NBA/LiCl) $C_{54}H_{80}N_6O_{12}$ (1005) 1012 (M+Li+)

Beispiel 22

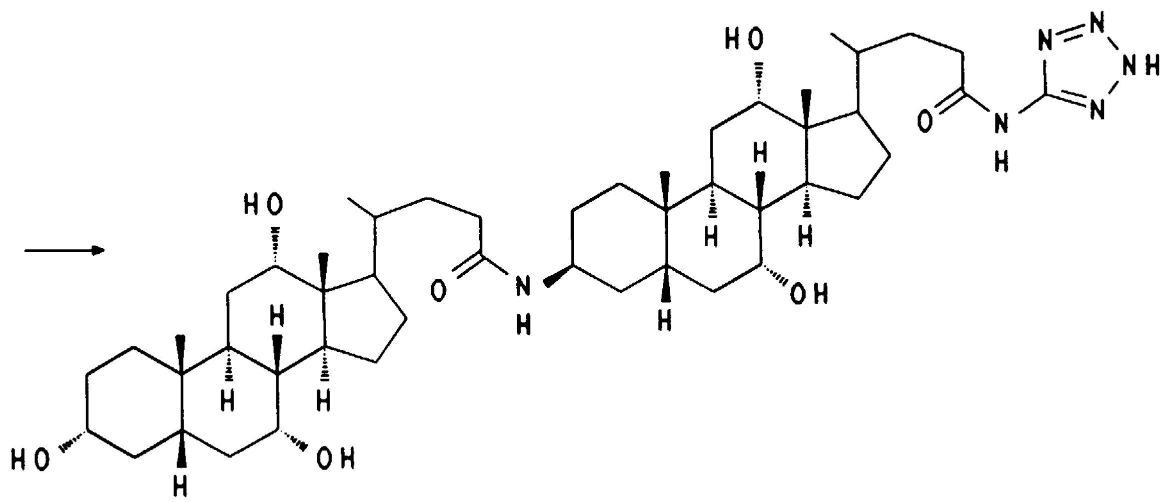

Zur Abspaltung der Formylgruppen werden 370 mg (0,37 mmol) Beispiel 21 in 20 ml EtOH gelöst, mit 2 ml 1N NaOH-Lösung versetzt und 6 Stunden bei Zimmertamperatur gerührt. Anschließend wird eingeengt, Wasser zugegeben und mit 1N HCl bis ~ pH 2 versetzt. Der Niederschlag wird abgesaugt, in Methanol gelöst, filtriert und erneut eingeengt. Es werden 170 mg (53 %) Produkt erhalten.
MS (FAB, 3-NBA/LiCl] $C_{49}H_{80}N_6O_7$ (865) 872 (M+Li+).

Die Beispiele 23 und 24 werden nach den für die Beispiele 21 und 22 beschriebenen Verfahren aus Triflormylcholsäure hergestellt.

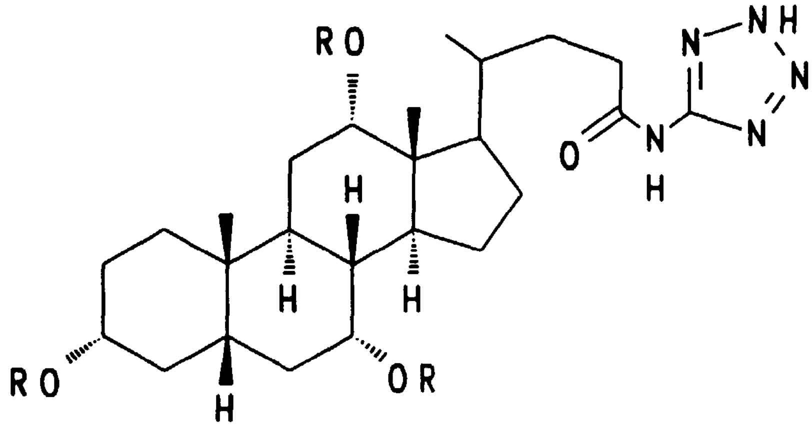

| Beispiel | R | MS (FAB, 3-NBA/LiCl) |
|---|---|---|
| 23 | -CHO | $C_{28}H_{41}N_5O_7$ (559) 566 (M+Li+) |
| 24 | H | $C_{25}H_{41}N_5O_4$ (475) 482 (M+Li+) |

Beispiel 25

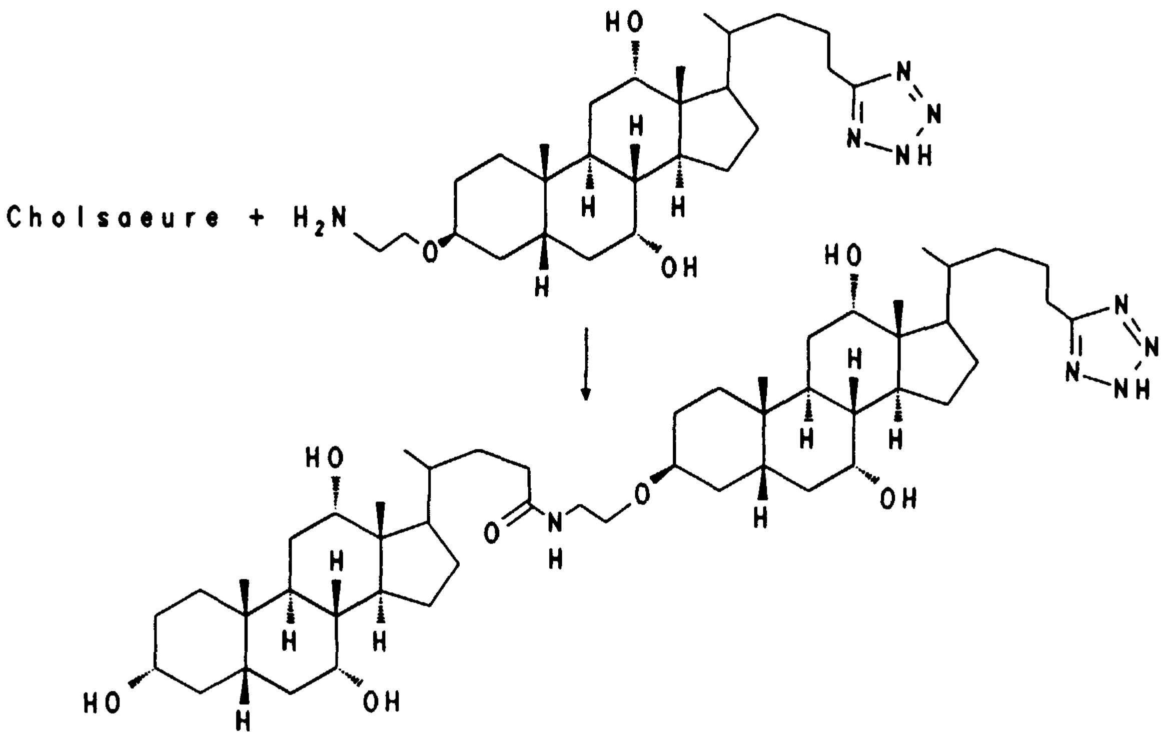


150 mg (0,31 mmol) Aminoverbindung (Beispiel 19), 130 mg (0,32 mmol) Cholsäure, 80 mg (0,64 mmol) Hydroxybenzotriazol und 65 mg (0,32 mmol) Dicyclohexylcarbondiimid werden in 20 ml THF 24 Stunden bei Zimmertemperatur gerührt. Das Reaktionsgemisch wird eingeengt und der Rückstand über Kieselgel chromatographiert ($CH_2Cl_2$/$MeOH/NEt_3$ 8:2:1). Es werden 250 mg (88 %) Produkt erhalten.

MS (FAB, 3-NBA/LiCl) $C_{51}H_{85}N_5O_7$ (880) 887 (M+Li+)

Die Beispiel 26 und 27 werden nach dem für Beispiel 25 beschriebenen Verfahren erhalten.

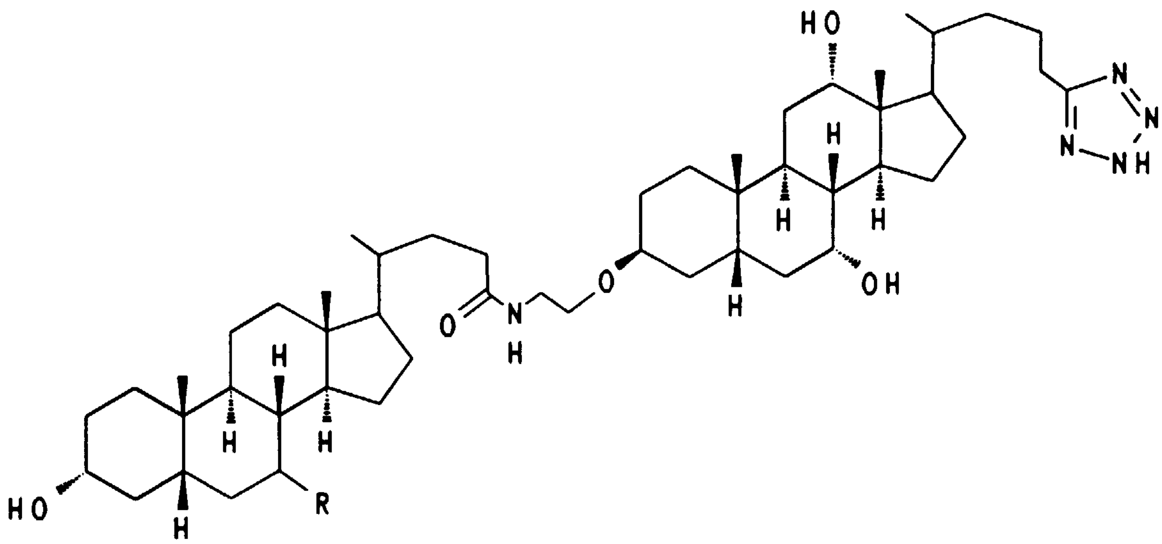

| Beispiel | R | MS (FAB, 3-NBA/LiCl) |
|---|---|---|
| 26 | α-OH | $C_{51}H_{85}N_5O_6$ (864) 865 (M+H+) |
| 27 | β-OH | $C_{51}H_{85}N_5O$ (864) 865 (M+H+) |

Beispiel 28

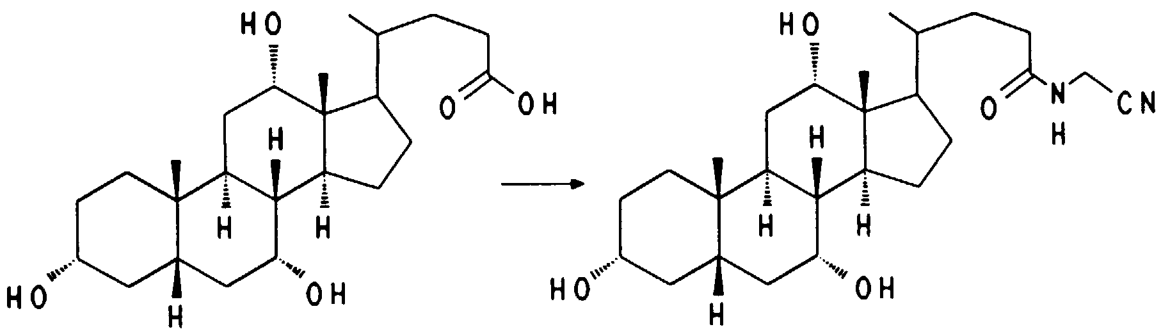

2,0 g (4,9 mmol) Cholsäure und 5 ml (36 mmol) Triethylamin werden in 150 ml THF gelöst und bei 0°C mit 1,5 ml (16 mmol) Chlorameisensäureethylester versetzt. Nach 15 Minuten werden 1,5 g (16 mmol) Aminoacetonitril Hydrochlorid zugegeben. Es wird 5 Stunden bei Zimmertemperatur gerührt. Der entstandene Niederschlag wird abfiltriert und die Lösung eingeengt. Nach Chromatographie des Rohprodukts (Chloroform/Methanol 17:3) werden 1,8 g Produkt (85 %) erhalten.
MS (FAB, 3-NBA/LiCl) $C_{26}H_{42}N_2O_4$ (446) 453 (M+Li+)

Beispiel 29

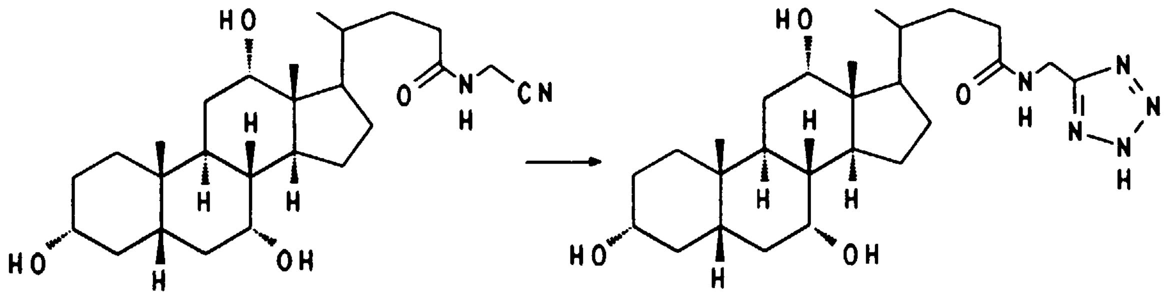

1,5 g (3,36 mmol) des Nitrils und 3,5 g (10,5 (10,5 mmol) Tributylzinnazid werden in 100 ml Toluol 24 Stunden unter Rückfluß erhitzt. Nach Beendigung der Reaktion wird im Vakuum eingeengt und der Rückstand über Kieselgel chromatographiert ($CHCl_3$/MeOH 7:3). Es werden 830 mg (56 %) Produkt erhalten.

MS (FAB, 3-NBA) $C_{26}H_{43}N_5O_4$ (489) 490 ($M+H^+$)

Die Beispiele 30 und 31 wurden nach den für die Beispiel 28 und 29 beschriebenen Verfahren hergestellt.

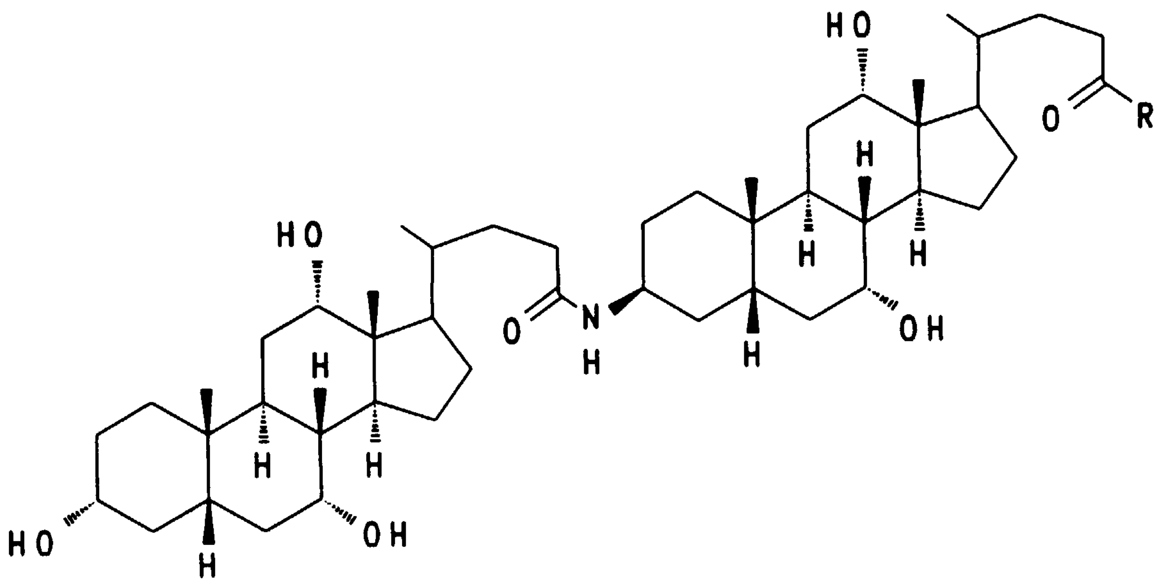

| Beispiel | R | MS (FAB, 3-NBA/LiCl) |
|---|---|---|
| 30 | 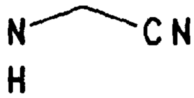 | $C_{50}H_{81}N_3O_7$ (835,6) 842,6 $(M+Li^+)$ |
| 31 | 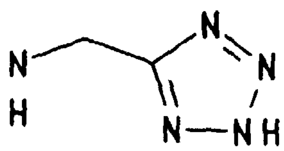 | $C_{50}H_{82}N_6O_7$ (878,6) 891,7 $(M+2Li-H^+)$ |

Tabelle 1 zeigt Meßwerte der Hemmung der [3H]-Taurocholataufnahme in Bürstensummembranvesikel des Ileums von Kaninchen. Angegeben sind die Quotienten aus den $IC_{50}$- bzw. $IC_{50Na}$-Werten der Referenzsubstant Taurochemodesoxycholat (TCDC) und der jeweiligen Testsubstanz.

Tabelle 1

| Verbindungun aus Beispiel | $\frac{IC_{50}\text{-TCDC } [\mu mol]}{IC_{50}\text{-Substanz } [\mu mol]}$ | $\frac{IC_{50Na}\text{TCDC } [\mu mol]}{IC_{50}\text{-Substanz } [\mu mol]}$ |
|---|---|---|
| | | |
| 4 | 0,28 | 0,38 |
| 14 | 1,20 | 1,35 |
| 16 | 0,26 | 0,25 |
| 22 | 1,66 | 1,35 |
| 24 | 0,97 | 1,27 |
| 27 | 0,26 | 0,25 |
| 29 | 0,46 | 0,48 |
| 31 | 1,39 | 1,24 |

**Patentansprüche**

**1.** Tetrazolgallensäurederivate der Formel I

$$G1 - X - G2 \qquad I$$

in der

G1 H oder einen Rest der Formel II bedeutet,

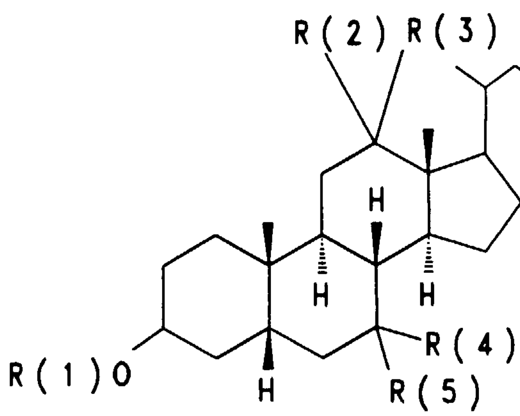

II

in der

R(1) H, einen Alkylrest oder Alkenylrest mit bis zu 10 C-Atomen, der verzweigt oder unverzweigt ist, einen Cycloalkylrest mit 3 bis 8 C-Atomen,
einen Benzylrest, der unsubstituiert oder 1- bis 3-fach substituiert ist mit F, Cl, Br, ($C_1$-$C_4$)-Alkyl oder ($C_1$-$C_4$)-Alkoxy,
einen Diphenylmethylrest, der unsubstituiert oder 1- bis 3-fach substituiert ist mit F, Cl, Br, ($C_1$-$C_4$)-Alkyl oder ($C_1$-$C_4$)-Alkoxy,
einen Triphenylmethylrest, der unsubstituiert oder 1- bis 3-fach substituiert ist mit F, Cl, Br, ($C_1$-$C_4$)-Alkyl oder ($C_1$-$C_4$)-Alkoxy,
einen $C_1$-$C_4$-Alkoxymethyl- oder einen Tetrahydroxylamylrest oder einen Rest

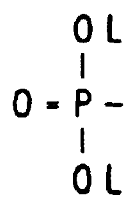 , 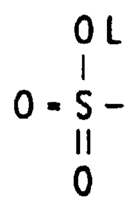 oder 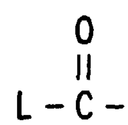

wobei L
H, einen Alkylrest oder Alkenylrest mit bis zu 10 C-Atomen, der verzweigt oder unverzweigt ist, einen Cycloalkylrest mit 3 bis 8 C-Atomen, einen Phenylrest, der unsubstituiert oder 1- bis 3-fach substituiert ist mit F, Cl, Br, ($C_1$-$C_4$)-Alkyl oder ($C_1$-$C_4$)-Alkoxy,
einen Benzylrest, der unsubstituiert oder 1- bis 3-fach substituiert ist mit F, Cl, Br, ($C_1$-$C_4$)-Alkyl oder ($C_1$-$C_4$)-Alkoxy bedeutet, darstellt,

R(2) bis R(5), wobei R(2) und R(3) bzw. R(4) und R(5) jeweils gemeinsam den Sauerstoff einer Carbonylgruppe oder einzeln und jeweils unabhängig voneinander H, -OL, -SL, -NHL, Tetrahydropyranyloxy oder $C_1$-$C_4$-Alkoxymethoxy bedeuten, wobei L die oben angegebene Bedeutung hat und

G2 einen Rest der allgemeinen Formel IV bedeutet

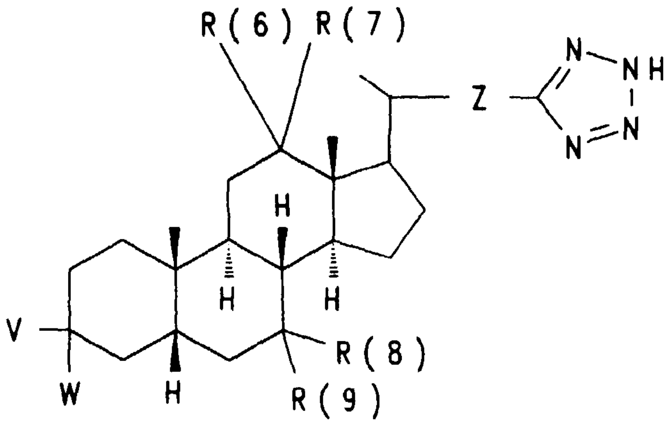

IV

wobei Z

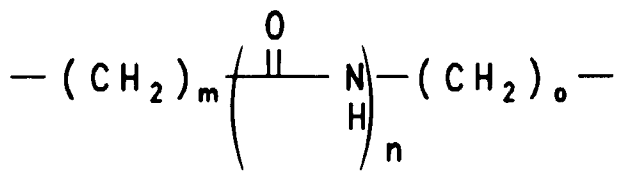

mit m = null bis 4, n = null oder 1 und o = null bis 4,

V

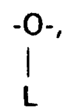

-N-, $-CH_2-$, $-CH_2CH_2-$

W H, oder falls V = $-CH_2-$, $-CH_2CH_2-$ ist, auch OH

und R(6) bis R(9) die unter R(2) bis R(5) angegebene Bedeutung haben und

X eine Brückengruppe oder eine kovalente Bindung bedeutet, wobei G1 und G2 beliebig über X verbunden sind.

**2.** Tetrazolgallensäurederivate der Formel I

G1 - X - G2      I

in der

G1 H oder einen Rest der Formel II bedeutet

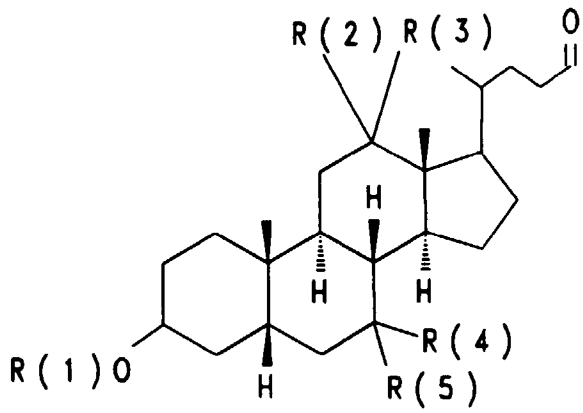

II

wobei

| | |
|---|---|
| R(1) | H, Formyl, Acetyl, Benzoyl, Methoxymethyl oder Tetrahyd ropyranyl, |
| R(2) bis R(5), | wobei R(2) und R(3) bzw. R(4) und R(5) jeweils gemeinsam den Sauerstoff einer Carbonylgruppe oder einzeln und unabhängig voneinander H, OH, O-Formyl, O-Acetyl, O-Benzoyl, Methoxymethoxy oder Tetrahydropyranyloxy bedeuten, |

X eine kovalente Bindung oder eine der folgenden Brückengruppen darstellt

$-\underset{H}{N}-(CH_2)_2-$ , $-\underset{H}{N}-(CH_2)_3-$ , $-\underset{H}{N}-(CH_2)_6-$ , $-\underset{H}{N}-(CH_2)_2-O-(CH_2)_2-$ ,

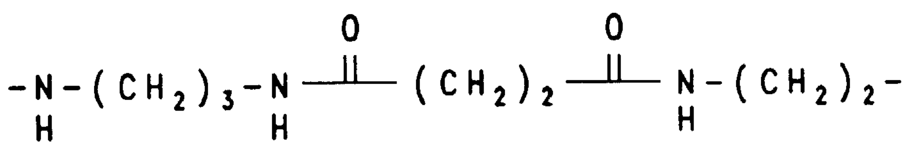

G2 einen Rest der Formel IV bedeutet

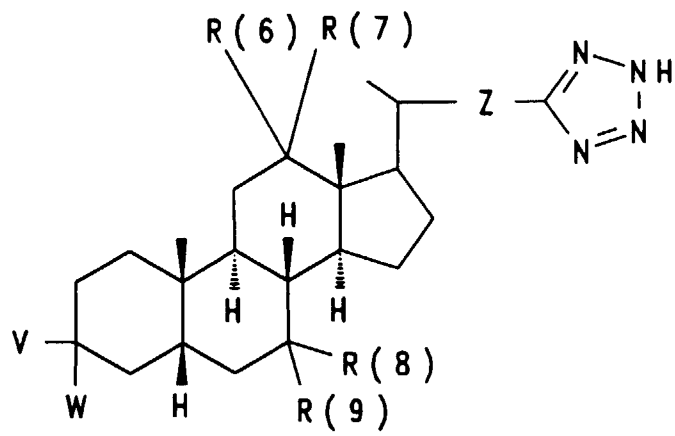

IV

wobei

V -O-,

-N-
H

W H

Z

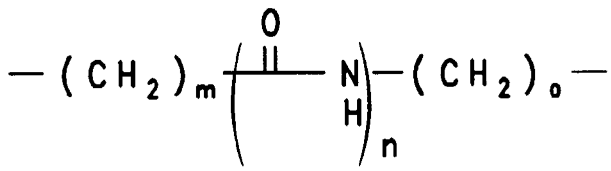

mit m = 1 bis 3, n = null oder 1 und o null, 1 oder 2 und

R(6) bis R(9) die vorstehend unter R(2) bis R(5) angegebene Bedeutung haben.

**3.** Arzneimittel enthaltend ein oder mehrere Tetrazolgallensäurederivate gemäß den Ansprüchen 1 oder 2.

**4.** Verwendung der Verbindungen der Formel I den Ansprüchen 1 oder 2 zur Herstellung eines Medikaments zur Behandlung von Cholestinstoffwechselstörungen.

**5.** Verwendung der Verbindungen der Formel I gemäß den Ansprüchen 1 oder 2 zur Herstellung eines Medikaments zur Behandlung von Hyperlipidämie.

## Claims

**1.** A tetrazole-bile acid derivative of the formula I

G1 - X - G2 I

in which

G1 is H or a radical of the formula II

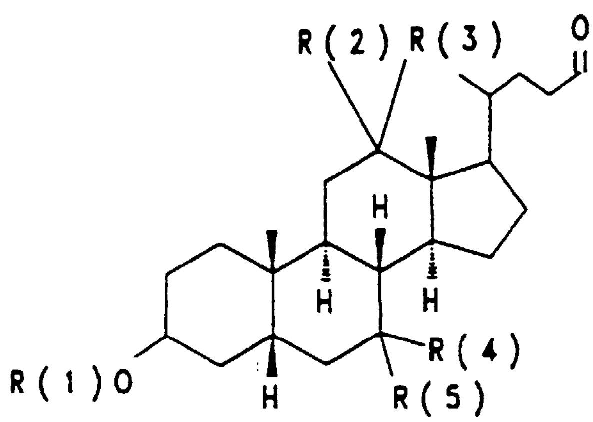

II

in which

R(1) is H, an alkyl radical or alkenyl radical having up to 10 carbon atoms, which is branched or unbranched,
a cycloalkyl radical having 3 to 8 carbon atoms,
a benzyl radical, which is unsubstituted or mono- to trisubstituted by F, Cl, Br, ($C_1$-$C_4$)-alkyl or ($C_1C_4$)- alkoxy,
a diphenylmethyl radical, which is unsubstituted or mono- to trisubstituted by F, Cl, Br, ($C_1$-$C_4$)-alkyl or ($C_1$-$C_4$)-alkoxy, a triphenylmethyl radical, which is unsubstituted or mono- to trisubstituted by F, Cl, Br, ($C_1$-$C_4$) -alkyl or ($C_1$-$C_4$)-alkoxy,
a $C_1$-$C_4$-alkoxymethyl or a tetrahydroxylamyl radical, or a radical

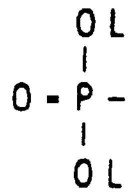 , 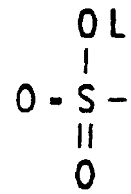 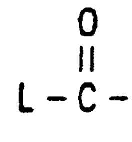

in which L is H, an alkyl radical or alkenyl radical having up to 10 carbon atoms, which is branched or unbranched, a cycloalkyl radical having 3 to 8 carbon atoms, a phenyl radical, which is unsubstituted or mono- to trisubstituted by F, Cl, Br, ($C_1$-$C_4$)-alkyl, ($C_1$-$C_4$)-alkoxy, or a benzyl radical, which is unsubstituted or mono- to trisubstituted by F, Cl, Br, ($C_1$-$C_4$)-alkyl or ($C_1$-$C_4$)-alkoxy,

R(2) to R(5), where R(2) and R(3) or R(4) and R(5) in each case together are the oxygen of a carbonyl group, or individually and in each case independently of one another are H, -OL, -SL, -NHL, tetrahydropyranyloxy or $C_1$-$C_4$-alkoxymethoxy, in which L has the abovementioned meaning, and

G2 is a radical of the formula IV

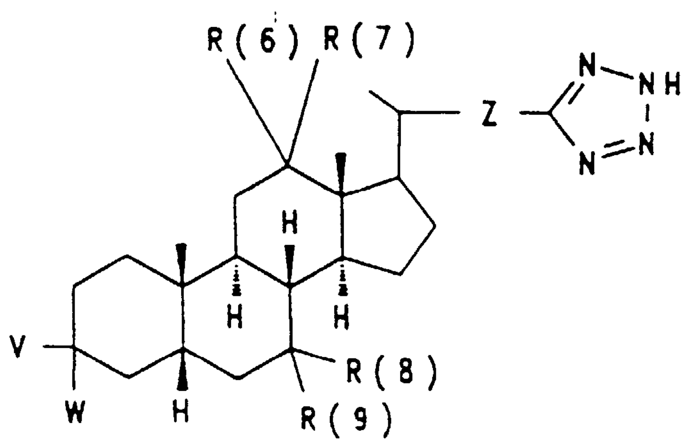

IV

in which Z is

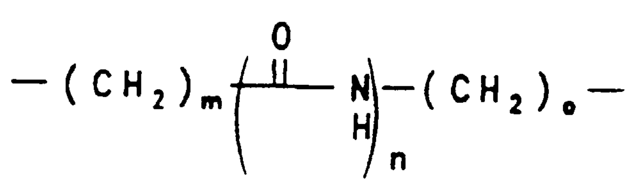

where m = zero to 4, n = zero or 1 and o = zero to 4,

V is

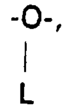

-N-, $-CH_2-$, $-CH_2CH_2-$

W is H or, if V is $-CH_2-$ or $-CH_2CH_2-$, also OH and R(6) to R(9) have the meaning given under R(2) to R(5) and

X is a bridge group or a covalent bond, and in which G1 and G2 can be bonded via X as desired.

**2.** A tetrazole-bile acid derivative of the formula I

$$G1 - X - G2 \qquad I$$

in which

G1 is H or a radical of the formula II

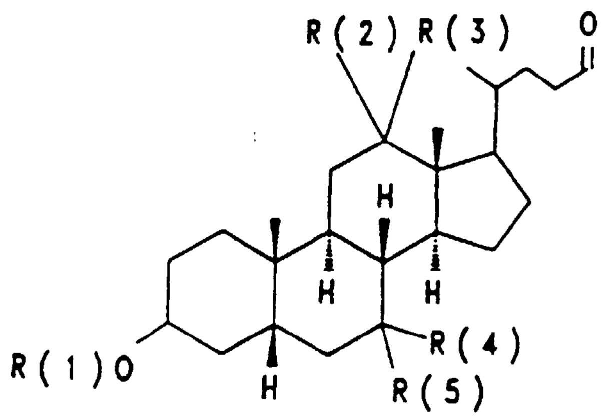

II

in which

| | |
|---|---|
| R(1) | is H, formyl, acetyl, benzoyl, methoxymethyl or tetrahydropyranyl, |
| R(2) to R(5), | where R(2) and R(3) or R(4) and R(5) in each case together are the oxygen of a carbonyl group, or individually and in each case independently of one another are H, OH, O-formyl, O-acetyl, O-benzoyl, methoxymethoxy or tetrahydropyranyloxy, |

X is a covalent bond or one of the following bridge groups

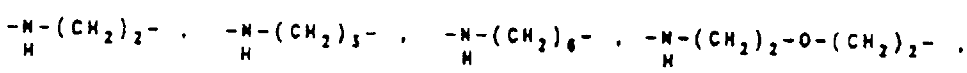

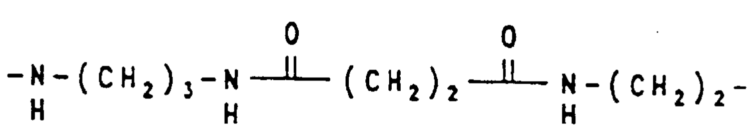

G2 is a radical of the formula IV

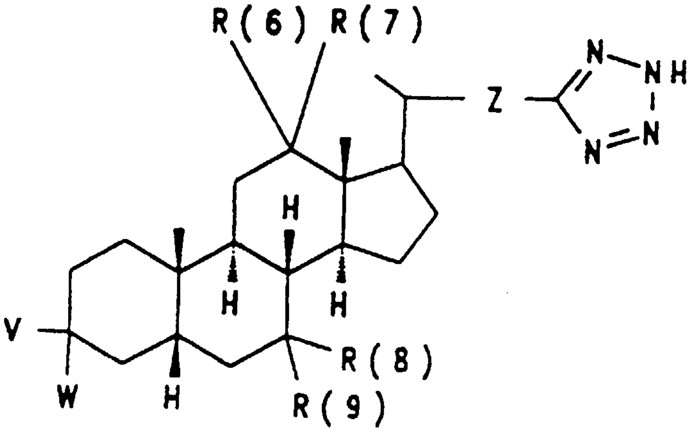

IV

in which

V is -O-,

-N-
H

W is H and

Z is

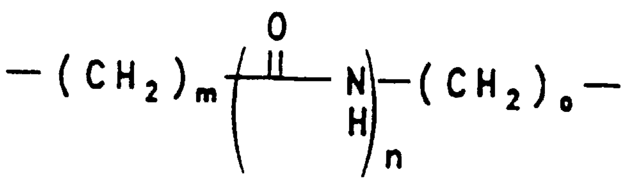

where m = 1 to 3, n = zero or 1 and o = zero, 1 or 2 and

R(6) to R(9) have the meaning given above under R(2) to R(5).

**3.** A medicament comprising one or more tetrazole-bile acid derivatives as claimed in claim 1 or 2.

**4.** The use of a compound of the formula I as claimed in claim 1 or 2 for the preparation of a medicament for treatment of disturbances in cholesterol metabolism.

**5.** The use of a compound of the formula I as claimed in claim 1 or 2 for the preparation of a medicament for treatment of hyperlipidaemia.

## Revendications

**1.** Dérivés tétrazole d'acides biliaires de formule I

G1 - X - G2 I,

dans laquelle

G1 signifie H ou un reste de formule II,

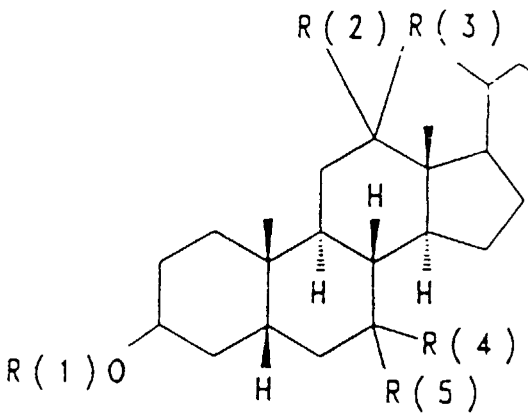

II

dans laquelle

R(1) représente H, un reste alkyle ou un reste alcényle comprenant jusqu'à 10 atomes de carbone, linéaire ou ramifié, un reste cycloalkyle à 3 à 8 atomes de carbone, un reste benzyle non substitué ou substitué 1 à 3 fois par F, Cl, Br, un groupe alkyle ($C_1$-$C_4$) ou alcoxy ($C_1$ -$C_4$), un reste diphénylméthyle non substitué ou substitué 1 à 3 fois par F, Cl, Br, un groupe alkyle ($C_1$-$C_4$) ou alcoxy ($C_1$-$C_4$), un reste triphénylméthyle non substitué ou substitué 1 à 3 fois par F, Cl, Br, un groupe alkyle ($C_1$-$C_4$) ou alcoxy ($C_1$-$C_4$), un reste alcoxy-($C_1$-$C_4$)méthyle ou tétrahydroxylamyle ou un reste

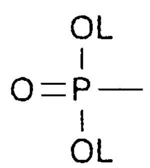 , 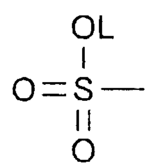 ou 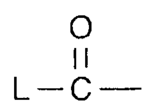

pour lequel L représente H, un reste alkyle ou un reste alcényle comprenant jusqu'à 10 atomes de carbone, linéaire ou ramifié, un reste cycloalkyle à 3 à 8 atomes de carbone, un reste phényle non substitué ou substitué 1 à 3 fois par F, Cl, Br, un groupe alkyle ($C_1$-$C_4$) ou alcoxy ($C_1$ -$C_4$), un reste benzyle non substitué ou substitué i à 3 fois par F, Cl, Br, un groupe alkyle ($C_1$-$C_4$) ou alcoxy ($C_1$-$C_4$),

R(2) à R(5) signifient, deux par deux, à savoir R(2) et R(3), respectivement R(4) et R(5), ensemble l'atome d'oxygène d'un groupe carbonyle, ou séparément et indépendamment les uns des autres, H, -OL, -SL, -NHL, un groupe tétrahydropyranyloxy ou alcoxy-(C-C4)-méthoxy, L ayant la signification donnée précédemment et

G2 signifie un reste de formule générale IV

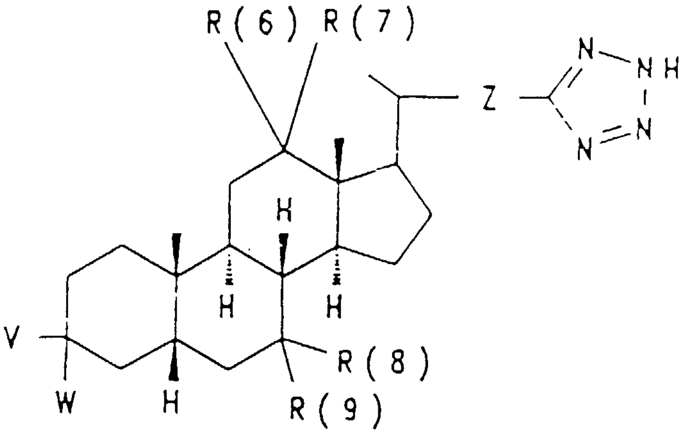

IV

dans laquelle

Z représente le groupe

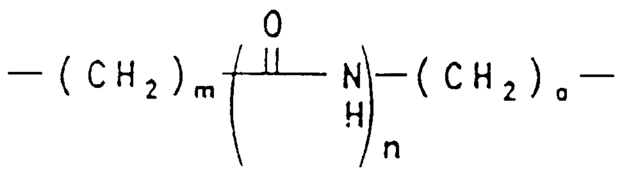

avec m = 0 à 4, n = 0 ou 1 et o = 0 à 4,

V -O-,

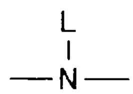,

$-CH_2-$, $-CH_2CH_2-$,

W H, ou si V = $-CH_2-$, $-CH_2CH_2-$, également OH,

et R(6) à R(9) ont les significations données pour R(2) à R(5) et

X signifie un groupe pontant ou une liaison covalente, G1 et G2 étant reliés par X de manière quelconque.

**2.** Dérivés tétrazole d'acides biliaires de formule I

G1 - X - G2     I,

dans laquelle

G1 signifie H ou un reste de formule II,

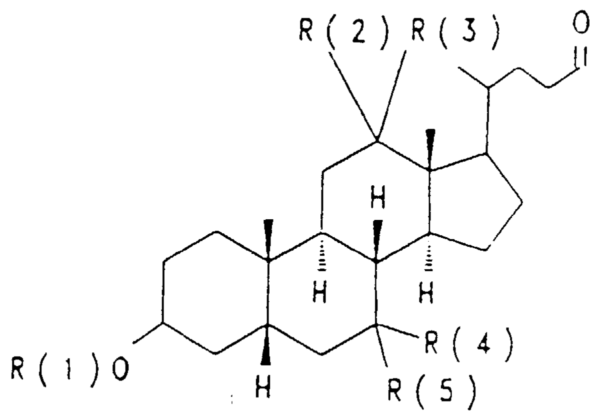

II

dans laquelle

R(1) représente H, un groupe formyle, acétyle, benzoyle, méthoxyméthyle ou tétrahydropyranyle,

R(2) à R(5) signifient, deux par deux, à savoir R(2) et R(3), respectivement R(4) et R(5), ensemble l'atome d'oxygène d'un groupe carbonyle, ou séparément et indépendamment les uns des autres, H, OH, O-formyle, O-acétyle, O-benzoyle, méthoxyméthoxy ou tétrahydropyranyloxy,

X signifie une liaison covalente ou un des groupes pontants suivants :

$-\underset{H}{N}-(CH_2)_2-$ , $-\underset{H}{N}-(CH_2)_3-$ , $-\underset{H}{N}-(CH_2)_6-$ , $-\underset{H}{N}-(CH_2)_2-O-(CH_2)_2-$ ,

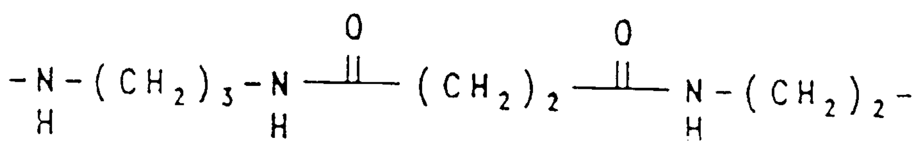

G2 signifie un reste de formule IV

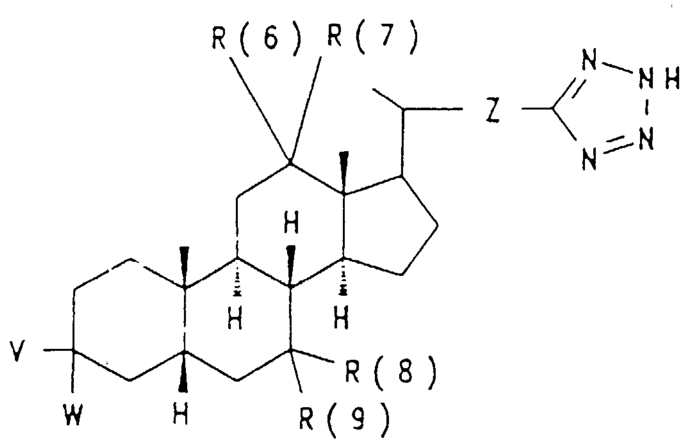

IV

dans laquelle

V représente -O-, -NH-,

W H,

Z le groupe

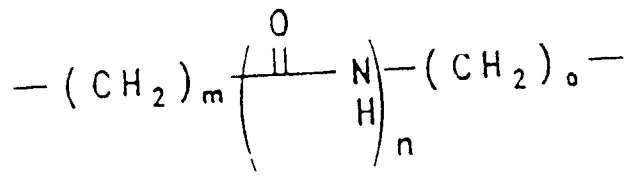

avec m = 1 à 3, n = 0 ou 1 et o = 0, 1 ou 2,

et R(6) à R(9) ont les mêmes significations que celles données précédemment pour R(2) à R(5).

**3.** Médicament contenant un ou plusieurs dérivés tétrazole d'acides biliaires selon la revendication 1 ou 2.

**4.** Utilisation des composés de formule I de la revendication 1 ou 2 pour la préparation d'un médicament pour le traitement de troubles du métabolisme du cholestérol.

**5.** Utilisation des composés de formule I selon la revendication 1 ou 2 pour la préparation d'un médicament pour le traitement de l'hyperlipidémie.